# EUROPEAN PATENT APPLICATION

(11) **EP 4 008 348 A1**
(43) Date of publication of application: **08.06.2022**
(21) Application number: 20846826.4
(22) Date of filing: 29.07.2020
(51) Int. Cl.: A61K 39/395, A61P 37/06, C07K 16/18, C07K 16/28, C07K 16/46, C12N 15/13

(54) **BISPECIFIC ANTIBODY**

(30) Priority: 30.07.2019 JP 2019139751
(71) Applicant: ONO Pharmaceutical Co., Ltd., Osaka-shi, Osaka 541-8526 (JP)
(72) Inventor: SHIBAYAMA, Shiro, Tsukuba-shi, Ibaraki 300-4247 (JP); SHIMBO, Takuya, Tsukuba-shi, Ibaraki 300-4247 (JP); TEZUKA, Tomoya, Tsukuba-shi, Ibaraki 300-4247 (JP); THROSBY, Mark, 3584CM Utrecht (NL); DE KRUIF, Cornelis Adriaan, 3584CM Utrecht (NL); VAN LOO, Pieter Fokko, 3584CM Utrecht (NL); KLOOSTER, Rinse, 3584CM Utrecht (NL)
(74) Representative: Grünecker Patent- und Rechtsanwälte PartG mbB
(86) International application number: PCT/JP2020/028972
(87) International publication number: WO 2021/020416

(57) **Abstract**

The present invention addresses the problem of providing a novel drug for preventing, inhibiting the progression of, inhibiting the recurrence of, or treating autoimmune diseases and the like. As the result of diligent research, the inventors of the present invention, focusing on PD-1/CD3 bispecific antibodies as substances capable of solving said problem, confirmed that said antibodies can be used in preparations for alleviating the occurrence of adverse reactions known as infusion reactions or cytokine release syndrome. The inventors also confirmed that said bispecific antibodies have the characteristic of permitting interaction between PD-1 and its ligand PD-L1, and discovered that such a characteristic contributes to potentiating or sustaining prevention of, inhibition of the progression of, inhibition of the recurrence of, or therapeutic effects upon autoimmune diseases.

## Description

### [Technical Field]

The present invention relates to a bispecific antibody capable of specifically binding to PD-1 and CD3, respectively (hereinafter, which may be abbreviated as a "PD-1/CD3 bispecific antibody") or an antibody fragment thereof (hereinafter, which may be collectively abbreviated as a "PD-1/CD3 bispecific antibody and the like"), and a pharmaceutical composition containing the same as an active ingredient, as well as pharmaceutical therapeutic use thereof.

### [Background Art]

PD-1 is an immunosuppressive receptor belonging to an immunoglobulin family and is a molecule having a function of suppressing the immune activation signals of T-cells activated by stimulation through an antigen receptor. From analysis of PD-1 knock-out mice or the like, it is known that PD-1 signals play important roles in suppression of autoimmune diseases such as autoimmune dilated cardiomyopathy, lupus-like syndrome, autoimmune encephalomyelitis, systemic lupus erythematosus, graft-versus-host disease, type I diabetes mellitus and rheumatoid arthritis. Accordingly, it is pointed out that a substance enhancing the PD-1 signal could be a prophylactic or therapeutic agent for autoimmune diseases.

It is known so far that there is a bispecific antibody recognizing PD-1 as a substance enhancing the PD-1 signal (Patent Literatures 1 to 3). This bispecific antibody is in the form which an antigen-recognition site of an antibody recognizing CD3, which is a member of a T-cell receptor complex, and an antigen-recognition site of an antibody recognizing PD-1 are linked to each other in genetic engineering method, and has an activity to enhance the inhibitory signal of PD-1 against the T-cell receptor complex by increasing the frequency of bringing PD-1 to the vicinity of the T-cell receptor complex. Furthermore, the Patent Literatures also state that the PD-1 bispecific antibody can be used for preventing or treating autoimmune diseases.

Incidentally, in protein formulations, the occurrence of adverse reactions called as infusion reaction or cytokine release syndrome immediately after administration have been concerned. Formulations in which such reactions are reduced or suppressed have been demanded.

In the PD-1/CD3 bispecific antibody of the present invention, cytokine production stimulation after administration, which is considered to be a cause of such adverse reactions, is sufficiently reduced, and therefore, it is expected to be a drug in which the occurrence of the concerned adverse reactions is suppressed. No bispecific antibody like this feature have been reported to date.

### [Citation List]

### [Patent Literature]

Patent Literature 1: International Publication No. WO2003/011911
Patent Literature 2: International Publication No. WO2004/072286
Patent Literature 3: International Publication No. WO2013/022091

### [Summary of Invention]

### [Technical Problem]

The object of the present invention is to provide a new pharmaceutical agent for preventing, suppressing the progression of symptoms of or the recurrence of or treating autoimmune diseases and the like.

### [Solution to Problem]

The inventors of the present invention diligently studied and focused on the PD-1/CD3 bispecific antibody of the present invention as a substance capable of solving the above-mentioned problem, and further verified that the PD-1/CD3 bispecific antibody could be an agent in which the occurrence of adverse reactions called as infusion reaction or cytokine release syndrome is reduced, and then completed the present invention.

Furthermore, the inventors of the present invention verified that the PD-1/CD3 bispecific antibody has a feature of allowing the interaction between PD-1 and PD-L1 as a ligand thereof, and found that such a feature contributes to enhancing or sustaining of the effect in preventing, suppressing the progression of symptoms of or the recurrence of or treating autoimmune diseases and the like of the PD-1/CD3 bispecific antibody.

That is, the present invention relates to the followings
[1] A bispecific antibody (hereinafter, which may be abbreviated as the "PD-1/CD3 bispecific antibody" as well as the above.) or an antibody fragment thereof, having a first arm specifically binding to PD-1 and a second arm specifically binding to CD3,
   wherein the first arm specifically binding to PD-1 has any one of VH selected from
   (A) a heavy chain variable region (hereinafter, the "heavy chain variable region" may be abbreviated as "VH") having
      (a) a complementary determining region 1 of the heavy chain variable region (hereinafter, the "complementary determining region 1 of the heavy chain variable region" may be abbreviated as "VH-CDR1") comprising the amino acid sequence set forth in SEQ ID No. 6,
      (b) a complementary determining region 2 of the heavy chain variable region (hereinafter, the "complementary determining region 2 of the heavy chain variable region" may be abbreviated as "VH-CDR2") comprising the amino acid sequence set forth in SEQ ID No. 7, and
      (c) a complementary determining region 3 of the heavy chain variable region (hereinafter, the "complementary determining region 3 of the heavy chain variable region" may be abbreviated as "VH-CDR3") comprising the amino acid sequence set forth in SEQ ID No. 8,
   (B) a VH having
      (a) a VH-CDR1 comprising the amino acid sequence set forth in SEQ ID No. 9,
      (b) a VH-CDR2 comprising the amino acid sequence set forth in SEQ ID No. 10, and
      (c) a VH-CDR3 comprising the amino acid sequence set forth in SEQ ID No. 11,
   (C) a VH having
      (a) a VH-CDR1 comprising the amino acid sequence set forth in SEQ ID No. 12,
      (b) a VH-CDR2 comprising the amino acid sequence set forth in SEQ ID No. 13, and
      (c) a VH-CDR3 comprising the amino acid sequence set forth in SEQ ID No. 14,
   (D) a VH having
      (a) a VH-CDR1 comprising the amino acid sequence set forth in SEQ ID No. 15,
      (b) a VH-CDR2 comprising the amino acid sequence set forth in SEQ ID No. 16, and
      (c) a VH-CDR3 comprising the amino acid sequence set forth in SEQ ID No. 17, and
   (E) a VH having
      (a) a VH-CDR1 comprising the amino acid sequence set forth in SEQ ID No. 18,
      (b) a VH-CDR2 comprising the amino acid sequence set forth in SEQ ID No. 19, and
      (c) a VH-CDR3 comprising the amino acid sequence set forth in SEQ ID No. 20, and
   wherein the second arm specifically binding to CD3 has a VH having
   (a) a VH-CDR1 comprising the amino acid sequence set forth in SEQ ID No.37,
   (b) a VH-CDR2 comprising the amino acid sequence set forth in SEQ ID No.38, and
   (c) a VH-CDR3 comprising the amino acid sequence set forth in SEQ ID No.39, and
   wherein one to five arbitrary amino acid residues may be substituted with other amino acids (preferably, conservative amino acids thereof) in any one or more of VH-CDRs selected from the VH-CDR1, VH-CDR2 and VH-CDR3 in the first arm specifically binding to PD-1, respectively, and/or one to five arbitrary amino acid residues may be substituted with other amino acids (preferably, conservative amino acids thereof) in any one or more of VH-CDRs selected from the VH-CDR1, VH-CDR2 and VH-CDR3 in the second arm specifically binding to CD3, respectively.
[2] The PD-1/CD3 bispecific antibody or antibody fragment thereof according to the preceding item [1], wherein the first arm specifically binding to PD-1 has any one of VH selected from
   (A) the VH having
      (a) the VH-CDR1 comprising the amino acid sequence set forth in SEQ ID No. 6,
      (b) the VH-CDR2 comprising the amino acid sequence set forth in SEQ ID No. 7, and
      (c) the VH-CDR3 comprising the amino acid sequence set forth in SEQ ID No. 8,
   (B) the VH having
      (a) the VH-CDR1 comprising the amino acid sequence set forth in SEQ ID No. 9,
      (b) the VH-CDR2 comprising the amino acid sequence set forth in SEQ ID No. 10, and
      (c) the VH-CDR3 comprising the amino acid sequence set forth in SEQ ID No. 11,
   (C) the VH having
      (a) the VH-CDR1 comprising the amino acid sequence set forth in SEQ ID No. 12,
      (b) the VH-CDR2 comprising the amino acid sequence set forth in SEQ ID No. 13, and
      (c) the VH-CDR3 comprising the amino acid sequence set forth in SEQ ID No. 14,
   (D) the VH having
      (a) the VH-CDR1 comprising the amino acid sequence set forth in SEQ ID No. 15,
      (b) the VH-CDR2 comprising the amino acid sequence set forth in SEQ ID No. 16, and
      (c) the VH-CDR3 comprising the amino acid sequence set forth in SEQ ID No. 17, and
   (E) the VH having
      (a) the VH-CDR1 comprising the amino acid sequence set forth in SEQ ID No. 18,
      (b) the VH-CDR2 comprising the amino acid sequence set forth in SEQ ID No. 19, and
      (c) the VH-CDR3 comprising the amino acid sequence set forth in SEQ ID No. 20, and
      wherein the second arm specifically binding to CD3 has the VH having
      (a) the VH-CDR1 comprising the amino acid sequence set forth in SEQ ID No.37,
      (b) the VH-CDR2 comprising the amino acid sequence set forth in SEQ ID No.38, and
      (c) the VH-CDR3 comprising the amino acid sequence set forth in SEQ ID No.39.
[3] The PD-1/CD3 bispecific antibody or antibody fragment thereof according to the preceding item [1] or [2], wherein
   (i) the VH of the first arm specifically binding to PD-1 has
      (a) the VH-CDR1 comprising the amino acid sequence set forth in SEQ ID No. 6,
      (b) the VH-CDR2 comprising the amino acid sequence set forth in SEQ ID No. 7, and
      (c) the VH-CDR3 comprising the amino acid sequence set forth in SEQ ID No. 8, and
   (ii) the VH of the second arm specifically binding to CD3 has
      (a) the VH-CDR1 comprising the amino acid sequence set forth in SEQ ID No. 37,
      (b) the VH-CDR2 comprising the amino acid sequence set forth in SEQ ID No. 38, and
      (c) the VH-CDR3 comprising the amino acid sequence set forth in SEQ ID No. 39.
[4] The PD-1/CD3 bispecific antibody or antibody fragment thereof according to the preceding item [1] or [2], wherein
   (i) the VH of the first arm specifically binding to PD-1 has
      (a) the VH-CDR1 comprising the amino acid sequence set forth in SEQ ID No. 9,
      (b) the VH-CDR2 comprising the amino acid sequence set forth in SEQ ID No. 10, and
      (c) the VH-CDR3 comprising the amino acid sequence set forth in SEQ ID No. 11, and
   (ii) the VH of the second arm specifically binding to CD3 has
      (a) the VH-CDR1 comprising the amino acid sequence set forth in SEQ ID No. 37,
      (b) the VH-CDR2 comprising the amino acid sequence set forth in SEQ ID No. 38, and
      (c) the VH-CDR3 comprising the amino acid sequence set forth in SEQ ID No. 39.
[5] The PD-1/CD3 bispecific antibody or antibody fragment thereof according to the preceding item [1] or [2], wherein
   (i) the VH of the first arm specifically binding to PD-1 has
      (a) the VH-CDR1 comprising the amino acid sequence set forth in SEQ ID No. 12,
      (b) the VH-CDR2 comprising the amino acid sequence set forth in SEQ ID No. 13, and
      (c) the VH-CDR3 comprising the amino acid sequence set forth in SEQ ID No. 14, and
   (ii) the VH of the second arm specifically binding to CD3 has
      (a) the VH-CDR1 comprising the amino acid sequence set forth in SEQ ID No. 37,
      (b) the VH-CDR2 comprising the amino acid sequence set forth in SEQ ID No. 38, and
      (c) the VH-CDR3 comprising the amino acid sequence set forth in SEQ ID No. 39.
[6] The PD-1/CD3 bispecific antibody or antibody fragment thereof according to the preceding item [1] or [2], wherein
   (i) the VH of the first arm specifically binding to PD-1 has
      (a) the VH-CDR1 comprising the amino acid sequence set forth in SEQ ID No. 15,
      (b) the VH-CDR2 comprising the amino acid sequence set forth in SEQ ID No. 16, and
      (c) the VH-CDR3 comprising the amino acid sequence set forth in SEQ ID No. 17, and
   (ii) the VH of the second arm specifically binding to CD3 has
      (a) the VH-CDR1 comprising the amino acid sequence set forth in SEQ ID No. 37,
      (b) the VH-CDR2 comprising the amino acid sequence set forth in SEQ ID No. 38, and
      (c) the VH-CDR3 comprising the amino acid sequence set forth in SEQ ID No. 39.
[7] The PD-1/CD3 bispecific antibody or antibody fragment thereof according to the preceding item [1] or [2], wherein
   (i) the VH of the first arm specifically binding to PD-1 has
      (a) the VH-CDR1 comprising the amino acid sequence set forth in SEQ ID No. 18,
      (b) the VH-CDR2 comprising the amino acid sequence set forth in SEQ ID No. 19, and
      (c) the VH-CDR3 comprising the amino acid sequence set forth in SEQ ID No. 20, and
   (ii) the VH of the second arm specifically binding to CD3 has
      (a) the VH-CDR1 comprising the amino acid sequence set forth in SEQ ID No. 37,
      (b) the VH-CDR2 comprising the amino acid sequence set forth in SEQ ID No. 38, and
      (c) the VH-CDR3 comprising the amino acid sequence set forth in SEQ ID No. 39.
[8] A bispecific antibody or an antibody fragment thereof, having a first arm specifically binding to PD-1 and a second arm specifically binding to CD3,
   wherein the first arm specifically binding to PD-1 has a VH having
   (a) a VH-CDR1 comprising an amino acid sequence represented by HYJ¹LH [wherein J¹ represents G (glycine) or A (alanine), and an alphabet represented by J¹ or other alphabets represent one-letter amino-acid abbreviations, respectively],
   (b) a VH-CDR2 comprising an amino acid sequence represented by WJ²NTNTU²NPTX²AQGFTG [wherein J² represents L (leucine) or I (isoleucine), U² represents E (glutamic acid) or G (glycine), X² represents F (phenylalanine) or Y (tyrosine), and an alphabet represented by J², U² or X² or other alphabets represent the same as the above, respectively], and
   (c) a VH-CDR3 comprising an amino acid sequence represented by GDJ³VVPTTIWNYYU³X³MZ³V [wherein J³ represents M (methionine) or L (leucine), U³ represents H (histidine) or Y (tyrosine), X³ represents F (phenylalanine) or Y (tyrosine), Z³ represents D (aspartic acid) or E (glutamic acid), and an alphabet represented by J³, U³, X³ or Z³, or other alphabets represent the same as the above, respectively], and
   wherein the second arm specifically binding to CD3 has the VH having
   (a) the VH-CDR1 comprising the amino acid sequence set forth in SEQ ID No. 37,
   (b) the VH-CDR2 comprising the amino acid sequence set forth in SEQ ID No. 38, and
   (c) the VH-CDR3 comprising the amino acid sequence set forth in SEQ ID No. 39.
[9] The PD-1/CD3 bispecific antibody or antibody fragment thereof according to the preceding item [8], wherein
   (a) J¹ represents G (glycine), J² represents L (leucine), U² represents E (glutamic acid), X² represents F (phenylalanine), J³ represents M (methionine), U³ represents H (histidine), X³ represents F (phenylalanine) and Z³ represents D (aspartic acid),
   (b) J¹ represents G (glycine), J² represents I (isoleucine), U² represents G (glycine), X² represents Y (tyrosine), J³ represents L (leucine), U³ represents H (histidine), X³ represents Y (tyrosine) and Z³ represents E (glutamic acid),
   (c) J¹ represents A (alanine), J² represents L (leucine), U² represents E (glutamic acid), X² represents Y (tyrosine), J³ represents M (methionine), U³ represents Y (tyrosine), X³ represents Y (tyrosine) and Z³ represents D (aspartic acid), or
   (d) J¹ represents A (alanine), J² represents L (leucine), U² represents E (glutamic acid), X² represents F (phenylalanine), J³ represents M (methionine), U³ represents H (histidine), X³ represents F (phenylalanine) and Z³ represents D (aspartic acid).
[10] The PD-1/CD3 bispecific antibody or antibody fragment thereof according to any one of the preceding items [1] to [9], wherein the framework region 1 (hereinafter, may be abbreviated as "FR1"), the framework region 2 (hereinafter, may be abbreviated as "FR2") and the framework region 3 (hereinafter, may be abbreviated as "FR3") in a framework region (hereinafter, the "framework region" may be abbreviated as "FR") of the VH of the first arm specifically binding to PD-1 correspond to amino acid sequences encoded by the germ-line V gene IGHV7-4-1 or gene thereof with somatic mutation(s), respectively.
[11] The PD-1/CD3 bispecific antibody or antibody fragment thereof according to the preceding item [10], wherein the framework region 4 (hereinafter, the "framework region 4" may be abbreviated as "FR4") in the VH of the first arm specifically binding to PD-1 comprises an amino acid sequence (excluding an amino acid sequence included in the VH-CDR3) encoded by the germ-line J gene JH6c or gene thereof with somatic mutation(s).
[12] The PD-1/CD3 bispecific antibody or antibody fragment thereof according to the preceding item [10] or [11], wherein the FR in the VH of the first arm specifically binding to PD-1 is encoded by the germ-line V gene IGHV7-4-1 which may have somatic mutation(s), and wherein the FR contains the FR1 in which in the amino acid sequence set forth in SEQ ID No. 21, by the somatic mutation(s), lysine at position 13 is or may be substituted with glutamine, alanine at position 16 is or may be substituted with valine, or lysine at position 19 is or may be substituted with methionine, or which is or may be carried out in arbitrary combination of a plurality thereof.
[13] The PD-1/CD3 bispecific antibody or antibody fragment thereof according to any one of the preceding items [10] to [12], wherein the FR in the VH of the first arm specifically binding to PD-1 is encoded by the germ-line V gene IGHV7-4-1 which may have somatic mutation(s), and wherein the FR contains the FR2 in which in the amino acid sequence set forth in SEQ ID No. 21, by the somatic mutation(s), valine at position 37 is or may be substituted with leucine.
[14] The PD-1/CD3 bispecific antibody or antibody fragment thereof according to any one of the preceding items [10] to [13], wherein the FR in the VH of the first arm specifically binding to PD-1 is encoded by the germ-line V gene IGHV7-4-1 which may have somatic mutation(s), and wherein the FR contains the FR3 in which in the amino acid sequence set forth in SEQ ID No. 21, by the somatic mutation(s), serine at position 77 is or may be substituted with threonine or cysteine at position 84 is or may be substituted with serine or asparagine, respectively, or which is or may be carried out in arbitrary combination of a plurality thereof.
[15] The PD-1/CD3 bispecific antibody or antibody fragment thereof according to any one of the preceding items [10] to [14], wherein the FR4 in the VH of the first arm specifically binding to PD-1 is encoded by the germ-line J gene JH6c which may have somatic mutation(s) (excluding the gene region encoding VH-CDR3), and wherein in the amino acid sequence (Trp-Gly-Lys-Gly-Thr-Thr^{∗}-Val-Thr-Val-Ser-Ser)(SEQ ID No. 41) of the FR4, lysine (Lys) is or may be substituted with glutamine or asparagine and/or threonine (Thr) marked with an asterisk is or may be substituted with leucine.
[16] The PD-1/CD3 bispecific antibody or antibody fragment thereof according to any one of the preceding items [1] to [15], wherein the VH of the first arm specifically binding to PD-1 comprises the amino acid sequence set forth in any one selected from SEQ ID No. 1, SEQ ID No. 2, SEQ ID No. 3, SEQ ID No. 4 and SEQ ID No. 5, or an amino acid sequence having an identity of at least 80%, 90%, 95%, 98% or 99% to the amino acid sequence of the same VH
[17] The PD-1/CD3 bispecific antibody or antibody fragment thereof according to any one of the preceding items [1] and [3] to [7], wherein the VH of the first arm specifically binding to PD-1 comprises the amino acid sequence set forth in any one selected from SEQ ID No. 1, SEQ ID No. 2, SEQ ID No. 3, SEQ ID No. 4 and SEQ ID No. 5.
[18] The PD-1/CD3 bispecific antibody or antibody fragment thereof according to any one of the preceding items [1] to [17], wherein the VH of the second arm specifically binding to CD3 comprises the amino acid sequence set forth in SEQ ID No. 36 or an amino acid sequence having an identity of at least 80%, 90%, 95%, 98% or 99% to the amino acid sequence of the same VH
[19] The PD-1/CD3 bispecific antibody or antibody fragment thereof according to any one of the preceding items [1] and [3] to [18], wherein the VH of the second arm specifically binding to CD3 comprises the amino acid sequence set forth in SEQ ID No. 36.
[20] The PD-1/CD3 bispecific antibody or antibody fragment thereof according to the preceding item [1], wherein the VH of the first arm specifically binding to PD-1 comprises the amino acid sequence set forth in any one selected from SEQ ID No. 1, SEQ ID No. 2, SEQ ID No. 3, SEQ ID No. 4 and SEQ ID No. 5, and the VH of the second arm specifically binding to CD3 comprises the amino acid sequence set forth in SEQ ID No. 36.
[21] A bispecific antibody or an antibody fragment thereof, having a first arm specifically binding to PD-1 and a second arm specifically binding to CD3, wherein a VH of the first arm specifically binding to PD-1 comprises the amino acid sequence set forth in any one selected from SEQ ID No. 1, SEQ ID No. 2, SEQ ID No. 3, SEQ ID No. 4 and SEQ ID No. 5, or an amino acid sequence having an identity of at least 80%, 90%, 95%, 98% or 99% to the amino acid sequence of the same VH, and a VH of the second arm specifically binding to CD3 comprises the amino acid sequence set forth in SEQ ID No. 36 or an amino acid sequence having an identity of at least 80%, 90%, 95%, 98% or 99% to the amino acid sequence of the same VH
[22] The PD-1/CD3 bispecific antibody or antibody fragment thereof according to the preceding item [1] or [3], wherein the VH of the first arm specifically binding to PD-1 comprises the amino acid sequence set forth in SEQ ID No. 1, and the VH of the second arm specifically binding to CD3 comprises the amino acid sequence set forth in SEQ ID No. 36.
[23] The PD-1/CD3 bispecific antibody or antibody fragment thereof according to the preceding item [1] or [4], wherein the VH of the first arm specifically binding to PD-1 comprises the amino acid sequence set forth in SEQ ID No. 2 and the VH of the second arm specifically binding to CD3 comprises the amino acid sequence set forth in SEQ ID No. 36.
[24] The PD-1/CD3 bispecific antibody or antibody fragment thereof according to the preceding item [1] or [5], wherein the VH of the first arm specifically binding to PD-1 comprises the amino acid sequence set forth in SEQ ID No. 3 and the VH of the second arm specifically binding to CD3 comprises the amino acid sequence set forth in SEQ ID No. 36.
[25] The PD-1/CD3 bispecific antibody or an antibody fragment thereof according to the preceding item [1] or [6], wherein the VH of the first arm specifically binding to PD-1 comprises the amino acid sequence set forth in SEQ ID No. 4 and the VH of the second arm specifically binding to CD3 comprises the amino acid sequence set forth in SEQ ID No. 36.
[26] The PD-1/CD3 bispecific antibody or antibody fragment thereof according to the preceding item [1] or [7], wherein the VH of the first arm specifically binding to PD-1 comprises the amino acid sequence set forth in SEQ ID No. 5 and the VH of the second arm specifically binding to CD3 comprises the amino acid sequence set forth in SEQ ID No. 36.
[27] The PD-1/CD3 bispecific antibody or antibody fragment thereof according to any one of the preceding items [1] to [26], wherein the first arm specifically binding to PD-1 and/or the second arm specifically binding to CD3 have/has a light chain variable region (hereinafter, the "light chain variable region" may be abbreviated as "VL") having
   (a) a complementary determining region 1 of light chain variable region (hereinafter, the "complementary determining region 1 of light chain variable region" may be abbreviated as "VL-CDR1") comprising the amino acid sequence set forth in SEQ ID No. 26,
   (b) a complementary determining region 2 of light chain variable region (hereinafter, the "complementary determining region 2 of light chain variable region" may be abbreviated as "VL-CDR2") comprising the amino acid sequence set forth in SEQ ID No. 27, and
   (c) a complementary determining region 3 of light chain variable region (hereinafter, the "complementary determining region 3 of light chain variable region" may be abbreviated as "VL-CDR3") comprising the amino acid sequence set forth in SEQ ID No. 28, respectively.
[28] The PD-1/CD3 bispecific antibody or antibody fragment thereof according to any one of the preceding items [1] to [27], wherein the first arm specifically binding to PD-1 and/or the second arm specifically binding to CD3 have/has a VL comprising the amino acid sequence set forth in SEQ ID No. 25, respectively.
[29] A bispecific antibody or an antibody fragment thereof, having a first arm specifically binding to PD-1 and a second arm specifically binding to CD3, wherein
   (A) the first arm specifically binding to PD-1 has a VH comprising the amino acid sequence set forth in any one selected from SEQ ID No. 1, SEQ ID No. 2, SEQ ID No. 3, SEQ ID No. 4 and SEQ ID No. 5, and a VL comprising the amino acid sequence set forth in SEQ ID No. 25, and
   (B) the second arm specifically binding to CD3 has a VH comprising the amino acid sequence set forth in SEQ ID No. 36, and a VL comprising the amino acid sequence set forth in SEQ ID No. 25.
[30] A bispecific antibody or an antibody fragment thereof, having a first arm specifically binding to PD-1 and a second arm specifically binding to CD3,
   wherein the first arm specifically binding to PD-1 cross-competes for (1) the binding to PD-1 with the first arm specifically binding to PD-1 having a VH comprising the amino acid sequence set forth in any one selected from SEQ ID No. 1, SEQ ID No. 2, SEQ ID No. 3, SEQ ID No. 4 and SEQ ID No. 5, and a VL comprising the amino acid sequence set forth in SEQ ID No. 25, or (2) the binding to PD-1 with a variable region of monoclonal antibody specifically binding to PD-1 having the same VH and VL.
[31] A bispecific antibody or an antibody fragment thereof, having a first arm specifically binding to PD-1 and a second arm specifically binding to CD3, wherein the binding to PD-1 with the first arm specifically binding to PD-1 is cross-competed by (1) the first arm specifically binding to PD-1 having a VH comprising the amino acid sequence set forth in any one selected from SEQ ID No. 1, SEQ ID No. 2, SEQ ID No. 3, SEQ ID No. 4 and SEQ ID No. 5, and a VL comprising the amino acid sequence set forth in SEQ ID No. 25, or (2) a variable region of monoclonal antibody specifically binding to PD-1 having the same VH and VL.
[32] The PD-1/CD3 bispecific antibody or antibody fragment thereof according to the preceding item [30] or [31], wherein the second arm specifically binding to CD3 further cross-competes for (1) the binding to CD3 with the second arm specifically binding to CD3 having the VH comprising the amino acid sequence set forth in SEQ ID No. 36, and the VL comprising the amino acid sequence set forth in SEQ ID No. 25 or (2) the binding to CD3 with a variable region of monoclonal antibody specifically binding to CD3 having the same VH and VL.
[33] The PD-1/CD3 bispecific antibody or antibody fragment thereof according to the preceding item [30] or [31], wherein the second arm specifically binding to CD3 has a VH having
   (a) the VH-CDR1 comprising the amino acid sequence set forth in SEQ ID No.37,
   (b) theVH-CDR2 comprising the amino acid sequence set forth in SEQ ID No.38, and
   (c) the VH-CDR3 comprising the amino acid sequence set forth in SEQ ID No.39.
[34] The PD-1/CD3 bispecific antibody or antibody fragment thereof according to any one of the preceding items [30], [31] and [33], wherein the VH in the second arm specifically binding to CD3 comprises the amino acid sequence set forth in SEQ ID No. 36 or an amino acid sequence having an identity of at least 80%, 90%, 95%, 98% or 99% to the amino acid sequence of the same VH
[35] The PD-1/CD3 bispecific antibody or antibody fragment thereof according to the preceding item [30] or [31], wherein the VH of the second arm specifically binding to CD3 comprises the amino acid sequence set forth in SEQ ID No. 36.
[36] The PD-1/CD3 bispecific antibody or antibody fragment thereof according to any one of the preceding items [30], [31] and [33] to [35], wherein the second arm specifically binding to CD3 has the VL having
   (a) the VL-CDR1 comprising the amino acid sequence set forth in SEQ ID No. 26,
   (b) the VL-CDR2 comprising the amino acid sequence set forth in SEQ ID No. 27, and
   (c) the VL-CDR3 comprising the amino acid sequence set forth in SEQ ID No. 28.
[37] The PD-1/CD3 bispecific antibody or antibody fragment thereof according to any one of the preceding items [30], [31] and [33] to [35], wherein the second arm specifically binding to CD3 has the VL comprising the amino acid sequence set forth in SEQ ID No. 25.
[38] The PD-1/CD3 bispecific antibody or antibody fragment thereof according to any one of the preceding items [1] to [37], wherein the PD-1/CD3 bispecific antibody is an IgG antibody.
[39] The PD-1/CD3 bispecific antibody or antibody fragment thereof according to the preceding item [38], wherein the IgG antibody in the preceding item [38] is an IgG₁ or IgG₄ antibody.
[40] The PD-1/CD3 bispecific antibody or antibody fragment thereof according to the preceding item [38], wherein the IgG antibody in the preceding item [38] is an IgG₁ antibody.
[41] The PD-1/CD3 bispecific antibody or antibody fragment thereof according to the preceding item [38], wherein the IgG antibody in the preceding item [38] is an IgG₄ antibody.
[42] The PD-1/CD3 bispecific antibody or antibody fragment thereof according to the preceding item [40], wherein the binding to Fc receptor of the IgG₁ antibody is eliminated or attenuated.
[43] The PD-1/CD3 bispecific antibody or antibody fragment thereof according to the preceding item [40] or [42], wherein in two heavy chain constant regions of the IgG₁ antibody in the preceding item [40], each leucine at position 235 according to the EU numbering system is substituted with glycine and/or each glycine at position 236 is substituted with arginine.
[44] The PD-1/CD3 bispecific antibody or antibody fragment thereof according to the preceding item [40], [42] or [43], wherein in a constant region of heavy chain having the VH of the first arm specifically binding to PD-1, both of leucine at position 351 and threonine at position 366 according to the EU numbering system are substituted with lysine, and in a constant region of heavy chain having the VH of the second arm specifically binding to CD3, leucine at position 351 is substituted with aspartic acid and leucine at position 368 is substituted with glutamic acid.
[45] The PD-1/CD3 bispecific antibody or antibody fragment thereof according to the preceding item [40], [42] or [43], wherein in the constant region of heavy chain having the VH of the first arm specifically binding to PD-1, leucine at position 351 according to the EU numbering system is substituted with aspartic acid and leucine at position 368 is substituted with glutamic acid, and in the constant region of heavy chain having the VH of the second arm specifically binding to CD3, both of leucine at position 351 and threonine at position 366 are substituted with lysine.
[46] The PD-1/CD3 bispecific antibody or antibody fragment thereof according to any one of the preceding items [40] and [42] to [45], wherein in two heavy chain constant regions of the IgG₁ antibody in any one of the preceding items [40] and [42] to [45], each lysine at position 447 according to the EU numbering system is deleted.
[47] The PD-1/CD3 bispecific antibody or antibody fragment thereof according to any one of the preceding items [38] to [46], wherein the binding to neonatal Fc receptor (hereinafter, may be abbreviated as "FcRn".) is eliminated or attenuated.
[48] The PD-1/CD3 bispecific antibody or antibody fragment thereof according to any one of the preceding items [40] and [42] to [47], wherein in two heavy chain constant regions of the IgG₁ antibody, (1) each methionine at position 252 according to the EU numbering system is substituted with glutamic acid, proline, arginine or aspartic acid, (2) each asparagine at position 434 according to the EU numbering system is substituted with leucine, and/or (3) each glutamine at position 438 according to the EU numbering system is substituted with glutamic acid.
[49] The PD-1/CD3 bispecific antibody or antibody fragment thereof according to any one of the preceding items [40] and [42] to [48], wherein methionine at position 252 according to the EU numbering system in two heavy chain constant regions of the IgG₁ antibody is substituted with aspartic acid.
[50] The PD-1/CD3 bispecific antibody or antibody fragment thereof according to the preceding item [48] or [49], wherein the half-life in blood of the IgG₁ antibody of the preceding item [48] or [49] is shortened compared to the original antibody without the same amino acid substitution(s).
[51] The PD-1/CD3 bispecific antibody or antibody fragment thereof according to the preceding item [48] or [49], wherein the half-life in blood of the IgG₁ antibody of the preceding item [48] or [49] is shortened at least 50%, at least 60%, at least 70%, at least 80% or at least 90%, compared to the original antibody without the same amino acid substitution(s).
[52] The PD-1/CD3 bispecific antibody or antibody fragment thereof according to the preceding item [41], wherein in two heavy chain constant regions of the IgG₄ antibody in the preceding item [41], each serine at position 228 according to the EU numbering system is substituted with proline.
[53] The PD-1/CD3 bispecific antibody or antibody fragment thereof according to any one of the preceding items [1] to [38], [40], [42], [43] and [45] to [51], wherein the heavy chain having the VH of the first arm specifically binding to PD-1 has a heavy chain constant region comprising the amino acid sequence set forth in any one selected from SEQ ID No. 23, SEQ ID No. 42, SEQ ID No. 43, SEQ ID No. 44, SEQ ID No. 45, SEQ ID No. 46 and SEQ ID No. 47.
[54] The PD-1/CD3 bispecific antibody or antibody fragment thereof according to any one of the preceding items [1] to [38], [40], [42], [43], [45] to [51] and [53], wherein the heavy chain having the VH of the second arm specifically binding to CD3 has a heavy chain constant region comprising the amino acid sequence set forth in any one selected from SEQ ID No. 24, SEQ ID No. 48, SEQ ID No. 49, SEQ ID No. 50, SEQ ID No. 51, SEQ ID No. 52 and SEQ ID No. 53.
[55] The PD-1/CD3 bispecific antibody or antibody fragment thereof according to any one of the preceding items [1] to [54], wherein the light chain having the VL of the first arm specifically binding to PD-1 and/or the light chain having the VL of the second arm specifically binding to CD3 has a light chain constant region comprising the amino acid sequence set forth in SEQ ID No. 29.
[56] A bispecific antibody or an antibody fragment thereof, having a first arm specifically binding to PD-1 and a second arm specifically binding to CD3, wherein
   (A) a heavy chain having the VH of the first arm specifically binding to PD-1 has a VH comprising the amino acid sequence set forth in any one selected from SEQ ID No. 1, SEQ ID No. 2, SEQ ID No. 3, SEQ ID No. 4 and SEQ ID No. 5, and a heavy chain constant region comprising the amino acid sequence set forth in any one selected from SEQ ID No. 23, SEQ ID No. 42, SEQ ID No. 43, SEQ ID No. 44, SEQ ID No. 45, SEQ ID No. 46 and SEQ ID No. 47,
   (B) a light chain having the VL of the first arm specifically binding to PD-1 has a VL comprising the amino acid sequence set forth in SEQ ID No. 25, and a light chain constant region comprising the amino acid sequence set forth in SEQ ID No. 29,
   (C) a heavy chain having the VH of the second arm specifically binding to CD3 has a VH comprising the amino acid sequence set forth in SEQ ID No. 36, and a heavy chain constant region comprising the amino acid sequence set forth in any one selected from SEQ ID No. 24, SEQ ID No. 48, SEQ ID No. 49, SEQ ID No. 50, SEQ ID No. 51, SEQ ID No. 52 and SEQ ID No. 53, and
   (D) a light chain having the VL of the second arm specifically binding to CD3 has a VL comprising the amino acid sequence set forth in SEQ ID No. 25, and a light chain constant region comprising the amino acid sequence set forth in SEQ ID No. 29.
[57] The PD-1/CD3 bispecific antibody or antibody fragment thereof according to any one of the preceding items [1] to [56], which specifically binds to PD-1 and CD3 expressed in a target cell, respectively.
[58] The PD-1/CD3 bispecific antibody or antibody fragment thereof according to any one of the preceding items [1] to [57], wherein the first arm specifically binding to PD-1 allows the interaction between PD-1 and PD-L1.
[59] The PD-1/CD3 bispecific antibody or antibody fragment thereof according to any one of the preceding items [1] to [58], wherein cytokine production during administration or within 24 hours after administration is sufficiently reduced.
[60] The PD-1/CD3 bispecific antibody or antibody fragment thereof according to any one of the preceding items [1] to [57], wherein the first arm specifically binding to PD-1 allows the interaction between PD-1 and PD-L1, and wherein cytokine production is sufficiently reduced.
[61] The PD-1/CD3 bispecific antibody or antibody fragment thereof according to the preceding item [59] or [60], wherein the cytokine includes at least IL-2, IFN-γ or TNF-α.
[62] The PD-1/CD3 bispecific antibody or antibody fragment thereof according to any one of the preceding items [1] to [61], wherein PD-1 is human PD-1, and CD3 is human CD3, respectively.
[63] The PD-1/CD3 bispecific antibody or antibody fragment thereof according to any one of the preceding items [1] to [62], wherein CD3 is CD3ε.
[64] The PD-1/CD3 bispecific antibody or antibody fragment thereof according to any one of the preceding items [1] to [63], wherein the PD-1/CD3 bispecific antibody is a monoclonal antibody.
[65] The PD-1/CD3 bispecific antibody or antibody fragment thereof according to any one of the preceding items [1] to [64], wherein the PD-1/CD3 bispecific antibody is an isolated antibody.
   [1-1] A pharmaceutical composition containing the PD-1/CD3 bispecific antibody or antibody fragment thereof according to any one of the preceding items [1] to [65] as an active ingredient.
   [1-2] The pharmaceutical composition according to the preceding item [1-1], which further contains a pharmaceutically acceptable carrier.
   [2-1] An agent for preventing, suppressing the progression of symptoms of or the recurrence of and/or treating autoimmune disease, containing the PD-1/CD3 bispecific antibody or antibody fragment thereof according to any one of the preceding items [1] to [65] as an active ingredient.
   [2-2] The agent according to the preceding item [2-1], wherein the autoimmune disease is Behcet's disease, systemic lupus erythematosus, chronic discoid lupus erythematosus, multiple sclerosis, systemic scleroderma, progressive systemic sclerosis, scleroderma, polymyositis, dermatomyositis, periarteritis nodosa (polyarteritis nodosa and microscopic polyangiitis), aortitis syndrome (Takayasu's arteritis), malignant rheumatoid arthritis, rheumatoid arthritis, juvenile idiopathic arthritis, spondylarthritis, mixed connective tissue disease, Castleman's disease, Sjogren's syndrome, adult Still's disease, vasculitis, allergic granulomatous vasculitis, hypersensitivity vasculitis, rheumatoid vasculitis, large vessel vasculitis, ANCA associated vasculitis (e.g., granulomatosis with polyangiitis and eosinophilic granulomatosis with polyangiitis), Cogan's syndrome, RS3PE syndrome, temporal arteritis, polymyalgia rheumatica, fibromyalgia, antiphospholipid antibody syndrome, eosinophilic fasciitis, IgG4-related disease (e.g., primary sclerosing cholangitis and autoimmune insulitis, etc.), Guillain-Barre syndrome, myasthenia gravis, chronic atrophic gastritis, autoimmune hepatitis, non-alcoholic steatohepatitis, primary biliary cirrhosis, Goodpasture's syndrome, rapidly progressive glomerulonephritis, lupus nephritis, megaloblastic anemia, autoimmune hemolytic anemia, pernicious anemia, autoimmune neutropenia, idiopathic thrombocytopenic purpura, Basedow disease (Graves' disease (hyperthyroidism)), Hashimoto disease, autoimmune adrenal insufficiency, primary hypothyroidism, Addison's disease (chronic hypoadrenocorticism), idiopathic Addison's disease, type I diabetes mellitus, slowly progressive type I diabetes mellitus (latent autoimmune diabetes in adult), focal scleroderma, psoriasis, psoriatic arthritis, bullous pemphigoid, pemphigus, pemphigoid, gestational herpes, linear IgA bullous dermatosis, acquired epidermolysis bullosa, alopecia areata, vitiligo, vitiligo vulgaris, neuromyelitis optica, chronic inflammatory demyelinating polyneuropathy, multifocal motor neuropathy, sarcoidosis, giant cell arteritis, amyotrophic lateral sclerosis, Harada disease, autoimmune optic neuropathy, idiopathic azoospermia, habitual abortion, inflammatory bowel disease (e.g., ulcerous colitis and Crohn's disease), celiac disease, ankylosing spondylitis, severe asthma, chronic urticaria, transplantation immunity, familial Mediterranean fever, eosinophilic chronic rhinosinusitis, dilated cardiomyopathy, systemic mastocytosis or inclusion body myositis.
   [2-3] An agent for preventing, suppressing the progression of symptoms of or the recurrence of and/or treating graft-versus-host disease (GVHD), containing the PD-1/CD3 bispecific antibody or antibody fragment thereof according to any one of the preceding items [1] to [65] as an active ingredient.
   [2-4] An agent for preventing, suppressing the progression of symptoms of or the recurrence of and/or treating type I diabetes mellitus, containing the PD-1/CD3 bispecific antibody or antibody fragment thereof according to any one of the preceding items [1] to [65] as an active ingredient, and being administered along with any one or more selected from an insulin formulation (e.g., human insulin, insulin glargine, insulin lispro, insulin detemir and insulin aspart, etc.), sulfonylurea agent (e.g., glibenclamide, gliclazide and glimepiride, etc.), quick-acting insulin secretion promoter (e.g., nateglinide etc.), biguanide preparation (e.g., metformin etc.), insulin resistance improving agent (e.g., pioglitazone etc.), α-glucosidase inhibitor (e.g., acarbose and voglibose, etc.), diabetic neuropathy therapeutic agent (e.g., epalrestat, mexiletine and imidapril, etc.), GLP-1 analog preparation (e.g., liraglutide, exenatide and lixisenatide, etc.) and DPP-4 inhibitor (e.g., sitagliptin, vildagliptin and alogliptin, etc.).
   [2-5] An agent for preventing, suppressing the progression of symptoms of or the recurrence of and/or treating multiple sclerosis, containing the PD-1/CD3 bispecific antibody or antibody fragment thereof according to any one of the preceding items [1] to [65] as an active ingredient, and being administered along with any one or more selected from a steroid agent (e.g., cortisone, cortisone acetate, hydrocortisone, hydrocortisone sodium phosphate, hydrocortisone sodium succinate, fludrocortisone acetate, prednisolone, prednisolone acetate, prednisolone sodium succinate, prednisolone butylacetate, prednisolone sodium phosphate, halopredone acetate, methylprednisolone, methylprednisolone acetate, methylprednisolone sodium succinate, triamcinolone, triamcinolone diacetate, triamcinolone acetonide, dexamethasone, dexamethasone acetate, dexamethasone valerate, dexamethasone cipecilate, dexamethasone palmitate, dexamethasone propionate, dexamethasone sodium phosphate, dexamethasone sodium metasulfobenzoate, parameterzone, parameterzone acetate, betamethasone, betamethasone dipropionate, betamethasone valerate, betamethasone acetate, betamethasone butyrate propionate and betamethasone sodium phosphate, etc.), interferon β-1a, interferon β-1b, glatiramer acetate, mitoxantrone, azathioprine, cyclophosphamide, cyclosporin, methotrexate, cladribine, adrenocorticotropic hormone (ACTH), corticotropin, mizoribine, tacrolimus, fingolimod and alemtuzumab.
   [2-6] An agent for preventing, suppressing the progression of symptoms of or the recurrence of and/or treating systemic lupus erythematosus, containing the PD-1/CD3 bispecific antibody or antibody fragment thereof according to any one of the preceding items [1] to [65] as an active ingredient, and being administered along with any one or more selected from a steroid agent (e.g., the steroid agents described in the preceding item [2-5]), immunosuppressive agent (e.g., cyclosporin, tacrolimus and fingolimod, etc.) and belimumab.
   [2-7] An agent for preventing, suppressing the progression of symptoms of or the recurrence of and/or treating rheumatoid arthritis, containing the PD-1/CD3 bispecific antibody or antibody fragment thereof according to any one of the preceding items [1] to [65] as an active ingredient, and being administered along with any one or more selected from a steroid agent (e.g., the steroid agents described in the preceding item [2-5]), anti-rheumatic agent (e.g., methotrexate, sulfasalazine, bucillamine, leflunomide, mizoribine and tacrolimus, etc.), anti-cytokine agent (e.g., infliximab, adalimumab, tocilizumab, etanercept, golimumab and certolizumab, etc.) and abatacept.
   [2-8] An agent for preventing, suppressing the progression of symptoms of or the recurrence of and/or treating autoimmune disease, containing the PD-1/CD3 bispecific antibody or antibody fragment thereof according to any one of the preceding items [1] to [65] as an active ingredient, and being administered along with any one or more of drugs described in the preceding items [2-4] to [2-7].
   [2-9] The agent for preventing, suppressing the progression of symptoms of or the recurrence of and/or treating each disease described in the preceding items [2-4] to [2-8], which is administered to the patient to which any one or more of drugs described in the preceding items [2-4] to [2-7] is/are administered.
   [2-10] The agent for preventing, suppressing the progression of symptoms of or the recurrence of and/or treating each disease described in the preceding items [2-4] to [2-8], which is administered after administration of any one or more of drugs described in the preceding items [2-4] to [2-7].
   [2-11] The agent for preventing, suppressing the progression of symptoms of or the recurrence of and/or treating each disease described in the preceding items [2-4] to [2-8], which is administered before administration of any one or more of drugs described in the preceding items [2-4] to [2-7].
   [3-1] An intravenous injection formulation containing the PD-1/CD3 bispecific antibody or antibody fragment thereof according to any one of the preceding items [1] to [65] and a pharmaceutically acceptable carrier.
   [3-2] The intravenous injection formulation according to the preceding item [3-1] for use in preventing, suppressing the progression of symptoms of or the recurrence of and/or treating autoimmune disease.
   [3-3] The intravenous injection formulation according to the preceding item [3-1] or [3-2] for use in drip infusion.
   [4-1] A method for preventing, suppressing the progression of symptoms of or the recurrence of and/or treating autoimmune disease, comprising administering an effective amount of the bispecific antibody or antibody fragment thereof according to any one of the preceding items [1] to [65] to a patient.
   [4-2] The bispecific antibody or antibody fragment thereof selected from any one of the preceding items [1] to [65] in use for preventing, suppressing the progression of symptoms of or the recurrence of and/or treating autoimmune disease.
   [4-3] Use of the bispecific antibody or antibody fragment thereof selected from any one of the preceding items [1] to [65] for manufacturing a pharmaceutical agent for preventing, suppressing the progression of symptoms of or the recurrence of and/or treating autoimmune disease.

### [Advantage Effects of Invention]

Since the inducibility of cytokine production of the PD-1/CD3 bispecific antibody of the present invention is reduced, it is expected that the occurrence of infusion reaction or cytokine release syndrome after administration is suppressed. Furthermore, it is expected that the feature of allowing the interaction between PD-1 and PD-L1 contributes to enhance or sustain the effect of preventing, suppressing the progression of symptoms of or the recurrence of and/or treating autoimmune disease.

### [Brief Description of the Drawings]

[Figure 1] It shows the respective amino acid sequences of VL and constant region of common light chain.
[Figure 2] It shows the respective CDR amino acid sequences in the VL of the common light chain.
[Figure 3] It shows the amino acid sequences encoded by germ-line V genes IGHV7-4-1 and IGHV3-33, respectively.
[Figure 4] It shows a sequence alignment among the VHs in the respective clones of antibodies specifically binding to PD-1 (hereinafter, may be abbreviated as "anti-PD-1 antibody") and germ-line genes IGHV7-4-1 and JH6c. In the amino acid sequences of the respective clones showed in this figure, "-" represents the same amino acid as that of the corresponding germ-line gene IGHV7-4-1 or JH6c, and abbreviations of amino acids represent amino acids different from that of the germ-line gene, respectively
[Figure 5] It shows the VH amino acid sequences of the respective anti-PD-1 antibody clones.
[Figure 6] It shows the respective CDR amino acid sequences in the VHs of the respective anti-PD-1 antibody clones.
[Figure 7] It shows the VH amino acid sequence of the CD3-2 clone as an antibody specifically binding to CD3 (hereinafter, may be abbreviated as "anti-CD3 antibody").
[Figure 8] It shows the respective CDR amino acid sequences in the VH of the CD3-2 clone as an anti-CD3 antibody.
[Figure 9] It shows the amino acid sequence of constant region in each heavy chain of the PD-1/CD3 bispecific monoclonal antibody.
[Figure 10] It shows the VH amino acid sequence of the anti-CD3 antibody clone 15C3 described in WO2005/118635. Note here that the underlined amino acid represents the 55th glycine which is converted into alanine in producing the CD3-1 clone.
[Figure 11] It shows the results of Biacore measurement verifying the binding activity to PD-1 and CD3 of the respective PD-1/CD3 bispecific monoclonal antibody clones, respectively.
[Figure 12] It shows flow cytometry verifying the simultaneous binding property to PD-1 and CD3 of the respective PD-1/CD3 bispecific monoclonal antibody clones.
[Figure 13] It shows flow cytometry verifying an influence on the PD-1/PD-L1 interaction of the respective PD-1/CD3 bispecific monoclonal antibody clones.
[Figure 14] It shows an effect on IFN-γ production from activated human T cells of the respective PD-1/CD3 bispecific monoclonal antibody clones. Note here that in this figure, "Ctrl" represents a control group.
[Figure 15] It shows the therapeutic effects of the respective PD-1/CD3 bispecific monoclonal antibody clones (PD1-1(Bi) and PD1-2(Bi)) in the experimental allergic encephalomyelitis mouse model (EAE model).
[Figure 16] It shows the therapeutic effects of the respective PD-1/CD3 bispecific monoclonal antibody clones (PD1-3(Bi) and PD1-4(Bi)) in the experimental allergic encephalomyelitis mouse model.
[Figure 17] It shows the therapeutic effects of the respective PD-1/CD3 bispecific monoclonal antibody clones (PD1-5(Bi) and PD1-6(Bi)) in the experimental allergic encephalomyelitis mouse model.
[Figure 18] It shows the effects on cytokine production from human peripheral blood mononuclear cell of the respective PD-1/CD3 bispecific monoclonal antibody clones.
[Figure 19] It shows the cross-competitive activity of PD1-5(Bi) against the bindings to PD-1 of the respective PD-1/CD3 bispecific monoclonal antibody clones.
[Figure 20] It shows the effect of the PD-1/CD3 bispecific monoclonal antibody in the mouse model transplanted with peripheral blood mononuclear cells. Marks presented by "^{∗}", "^{∗∗}" and "^{∗∗∗}" in this figure presents the significance compared to control at p <0.05, p <0.01 and p <0.001, by t-test, respectively.
[Figure 21] It shows the effect of the PD-1/CD3 bispecific monoclonal antibody in the mouse model producing antibody. Marks presented by "^{∗}" and "^{∗∗}" in this figure presents the significance compared to control at p <0.05 and p <0.01, by t-test, respectively.
[Figure 22] It shows the therapeutic effect of the PD-1/CD3 bispecific monoclonal antibody in the spontaneous diabetes model mouse model.
[Figure 23] It shows the results in the human FcRn binding properties to of the PD-1/CD3 bispecific monoclonal antibodies, PD1-5(Bi) and Mut1 to Mut3 clones, measured by Biacore (Registered Trademark).
[Figure 24] It shows the results in the human FcRn binding properties of the PD-1/CD3 bispecific monoclonal antibodies, Mut4 to Mut6 clones, measured by Biacore (Registered Trademark).
[Figure 25] It shows the results in the mouse FcRn binding properties to of the PD-1/CD3 bispecific monoclonal antibodies, PD1-5(Bi), Mut1 and Mut4 clones, measured by Biacore (Registered Trademark).
[Figure 26] It shows the therapeutic effect of the PD-1/CD3 bispecific monoclonal antibody Mut4 clone in the EAE model. Marks presented by "^{∗∗}" in this figure presents the significance compared to control at p <0.05 by Wilcoxon rank sum test.

### [Description of Embodiments]

PD-1 (Programmed Cell Death-1) in human is a membrane-type protein composed of the amino acid sequence represented by GenBank accession number NP_005009. In the present specification, the term "PD-1", unless specifically defined otherwise, may be used as a meaning including all of isoforms thereof and further variants thereof in which an epitope of the "first arm specifically binding to PD-1" of the present invention has been conserved. In the present invention, PD-1 is preferably human PD-1.

CD3 is the membrane-type protein forming T-cell receptor complex by association with T-cell receptor. In the present specification, the term "CD3", unless specifically defined otherwise, may be used as a meaning including subtypes (ε, δ, γ and ζ subtype) and further variants thereof in which an epitope of the "second arm specifically binding to CD3" of the present invention has been conserved. In the present invention, CD3 is preferably CD3ε or human CD3, and more preferably human CD3ε.

In the present specification, the term "isolate" means becoming a single substantially pure component by being identified, separated and/or purified from impurities containing a plurality of or myriad number of components extracted from host cells.

In the present specification, the term "monoclonal antibody" means an antibody obtained from a substantially homogeneous antibody group binding to the same specific antigen.

In this specification, the term "bispecific antibody" means an antibody having binding specificity to two different antigen molecules or epitopes in one molecule, and the term "bispecific monoclonal antibody" means a bispecific antibody obtained from a substantially homogeneous antibody group.

The present invention relates to a bispecific antibody capable of specifically binding to PD-1 and CD3 (in the present specification, may be abbreviated as a "PD-1/CD3 bispecific antibody"). In the present invention, the PD-1/CD3 bispecific antibody is preferably a PD-1/CD3 bispecific monoclonal antibody, and more preferably an isolated PD-1/CD3 bispecific monoclonal antibody, and furthermore preferably an isolated human PD-1/human CD3 bispecific monoclonal antibody. Herein, the "isolated human PD-1/human CD3 bispecific monoclonal antibody" means an isolated bispecific monoclonal antibody for human PD-1 and human CD3.

Herein, examples of forms of the bispecific antibodies include diabody, bispecific sc(Fv)₂, bispecific minibody, bispecific F(ab')₂, bispecific hybrid antibody, covalent diabody (bispecific DART), bispecific (FvCys)₂, bispecific F(ab'-zipper)₂, bispecific (Fv-zipper)₂, bispecific three-chain antibody and bispecific mAb², Addbody (registered trade mark) and Mirabody (registered trade mark) and the like.

The diabody is a dimer of single-chain peptides in which a VH and VL recognizing different antigens are linked to each other with a peptide linker (Proc. Natl. Acad. Sci. USA, 1993, Vol. 90, No. 14, pp. 6444-6448).

The bispecific sc(Fv)₂ is a low-molecular antibody modified such that two pairs of VH/VL of two antibodies recognizing different antigens are linked to each other with a peptide linker to form a continuous single chain (see J. Biological Chemistry, 1994, 269: pp. 199-206).

The bispecific F(ab')₂ is a low-molecular antibody in which Fab' fragments of antibodies recognizing two different antigens were covalently bonded through a disulfide bond or the like.

The bispecific minibody is a low-molecular antibody in which the low-molecular antibody fragments modified in such a manner that the constant region CH3 domains of antibody are linked to scFv recognizing different antigens, respectively, was covalently bonded by disulfide bonds or the like on the CH3 domains (see Biochemistry, 1992, Vo. 31, No .6, pp. 1579-1584).

The bispecific hybrid antibody is an intact antibody in which the heavy chain/light chain complexes recognizing two different antigens were covalently bound each other by a disulfide bond or the like.

In the present invention, the form of the bispecific antibody is preferably a bispecific hybrid antibody.

The bispecific hybrid antibody can be produced from a hybridoma produced by, for example, hybrid hybridoma method (see US4474893). Alternatively, the bispecific hybrid antibody can be produced by having a mammal animal cell co-express four kinds of cDNAs encoding a heavy chain and light chain of antibody recognizing different antigens, respectively, and secrete it.

The monoclonal antibodies used in the present invention can be produced by hybridoma method (see, e.g., Kohler and Milstein et al., Nature (1975), Vol. 256, p.495-97, Hongo et al., Hybridoma (1995), Vol. 14, No. 3, pp.253-260, Harlow et al., Antibodies: A Laboratory Manual, (Cold Spring Harbor Laboratory Press (1988), Vol. 2) and Hammerling et al., Monoclonal Antibodies and T-Cell Hybridomas, pp.563-681 (Elsevier, N.Y., 1981)), recombinant DNA method (see, e.g., US4816567), phage display method (see, e.g., Ladner et al., US5223409, US5403484 and US5571698, Dower et al., US5427908 and US5580717, McCafferty et al., US5969108 and US6172197, and Griffiths et al., US5885793, US6521404, US6544731, US6555313, US6582915 and US6593081).

An antibody or a monoclonal antibody, when being administered to human, can be produced in a form of a chimeric antibody, humanized antibody or complete human antibody in order to reduce or eliminate the antigenicity thereof.

The term "chimeric antibody" means an antibody of which variable region sequence and constant region sequence are derived from different mammalian. Examples of the chimeric antibodies include an antibody of which the variable region sequence is derived from a mouse antibody and the constant region sequence is derived from a human antibody. The chimeric antibody can be produced by linking a gene encoding an antibody variable region isolated from antibody-producing hybridomas isolated by the above-mentioned hybridoma method, recombinant DNA method or phage display method by well-known techniques to a gene encoding the constant region of human-derived antibody using well-known methods (see, e.g., Cabilly et al., US4816567).

The term "humanized antibody" means an antibody of which complementarity determining region (CDR) sequences derived from a germ line of other mammals such as mice were grafted into human framework region sequences. The humanized antibody can also be produced by linking genes encoding the CDRs of antibody isolated from antibody-producing hybridomas isolated by the above-mentioned method, by well-known techniques, to a gene encoding a framework region of the human-derived antibody using well-known methods (see, e.g., Winter, US5225539 and US5530101; Queen et al., US5585089 and US6180370).

The term "human antibody" or "complete human antibody" means an antibody in which both of variable regions composed of framework regions and CDRs and constant regions are derived from human germline immunoglobulin sequences. The human antibody to be used in the present invention can be produced by a method using mice transformed to produce a human antibody, for example, Humab mice (see, e.g., Lonberg and Kay et al., US5545806, US5569825, US5625126, US5633425, US5789650, US5877397, US5661016, US5814318, US5874299 and US5770429), KM mice (see, e.g., Ishida et al., WO2002/43478), Xeno mice (see, e.g., US5939598, US6075181, US6114598, US6150584 and US6162963), or Tc mice (see, e.g., Tomizuka et al., Proc. Natl. Acad. Sci. USA (2000), pp.722-727). Alternatively, the human antibody can also be prepared using SCID mice into which human immune cells have been reconstructed such that a human antibody response is made upon immunization (see, e.g., Wilson et al., US5476996 and US5698767). Furthermore, the human antibody to be used in the present invention can also be produced by the above-mentioned phage display method.

In the present specification, the term "antibody fragment" of the PD-1/CD3 bispecific antibody is a part of the full-length antibody and is an antibody having an antigen binding part to PD-1 and an antigen binding part to CD3. Examples thereof include F(ab')₂ and the like. Herein, the antigen binding part means a minimum unit of an antibody which can bind to an antigen thereof, for example, it is composed of three CDRs in the respective VH and VL and framework regions for arranging CDRs such that the target antigen can be recognized by combination of those CDRs.

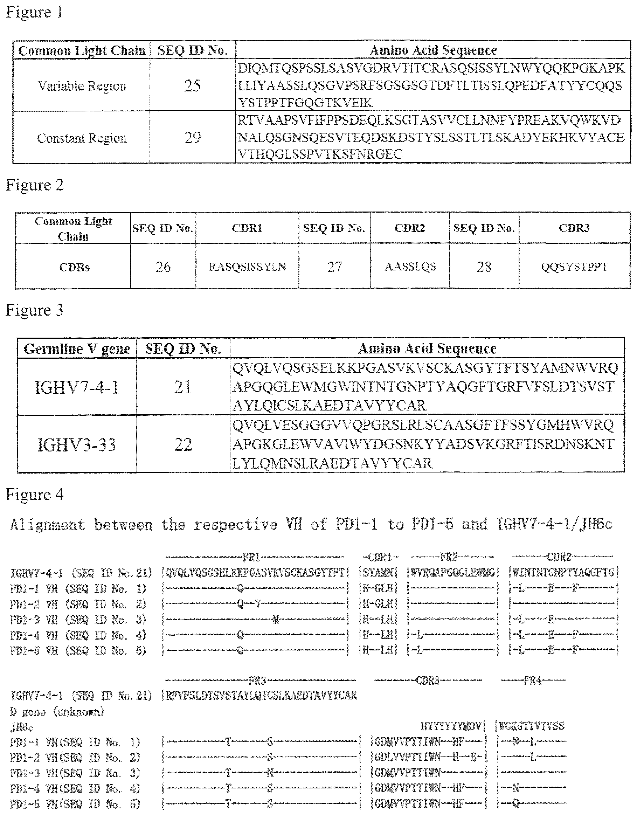

In the present specification, the term "common light chain" means a light chain which can associate with two or more different heavy chains and can exhibit the binding ability to each antigen (De Wildt RM et al., J. Mol. Biol. (1999), Vol. 285, pp.895-901, De Kruif et al., J. Mol. Biol. (2009), Vol. 387, pp.548-58, WO2004/009618, WO2009/157771 and WO2014/051433). Preferable examples of such common light chains include a light chain encoded by human κ light chain IgVκ1-39^{∗}01/IGJκ1^{∗}01 (nomenclatures of IMGT database) germ-line gene (hereinafter, may be abbreviated as "IGVK1-39/JK1 common light chain"). More preferable examples thereof include a light chain having a VL having the CDR1 comprising the amino acid sequence set forth in SEQ ID No. 26, the CDR2 comprising the amino acid sequence set forth in SEQ ID No. 27, and the CDR3 comprising the amino acid sequence set forth in SEQ ID No. 28, and further preferable examples thereof include a light chain having the VL comprising the amino acid sequence set forth in SEQ ID No. 25. Furthermore, preferable examples of the constant regions of common light chain include the light chain constant region comprising the amino acid sequence set forth in SEQ ID No. 29. The respective amino acid sequences of the VL and the constant region of common light chain to be used in the present invention are shown in Figure 1, and the respective amino acid sequence of CDRs of the variable region is shown in Figure 2.

In the present specification, the term "isotype" means the antibody class (e.g., IgM or IgG) which is encoded by heavy chain constant region genes. The isotype for the PD-1/CD3 bispecific antibody of the present invention is preferably IgG, and more preferably, IgG₁ or IgG₄. Herein, the IgG₁ is preferably of which the binding to an Fc receptor (e.g., Fc-gamma receptor) was eliminated or decreased. Specifically, an IgG₁ antibody of which the binding to the Fc receptor is eliminated or decreased can be obtained by substituting, deleting or inserting arbitrary amino acids of the heavy chain constant region thereof. Examples thereof include an antibody in which each leucine at position 235 according to the EU numbering system was substituted with glycine and/or each glycine at position 236 was substituted with arginine on two heavy chain constant regions or hinge regions thereof. Furthermore, in order to reduce the heterogeneity of antibody, an antibody in which an amino acid at C-terminal, for example, each lysine at position 447 according to the EU numbering system has been deleted is preferable. Furthermore, in order to shorten the half-life in blood, in particular, those of which the binding to FcRn receptor was eliminated or attenuated are also preferable. Specifically, it can be eliminated or attenuated by substitution or deletion of amino acids at the binding site to FcRn receptor of the heavy chain constant region, but in the case of IgG₁ antibody, examples of such antibodies include those of which (1) each methionine at position 252 according to the EU numbering system was substituted with glutamic acid, proline, arginine or aspartic acid, (2) each asparagine at position 434 according to the EU numbering system was substituted with leucine, and/or (3) each glutamine at position 438 according to the EU numbering system was substituted with glutamic acid. In the case of the IgG₄ bispecific antibody, in order to suppress the swapping in an antibody molecule, a variant in which an arbitrary amino acid in the heavy chain constant region thereof was substituted, deleted or inserted is preferable. Preferable examples thereof include an antibody of which serine at position 228 according to the EU numbering system, located in the hinge region, was substituted with proline. Note here that in the present specification, amino acid positions assigned to CDRs and framework regions in variable regions of antibody may be specified according to Kabat's numbering system (see Sequences of Proteins of Immunological Interest (National Institute of Health, Bethesda, Md., 1987 and 1991)). Furthermore, amino acids in the constant region are indicated according to the EU numbering system based on Kabat's amino acid positions (see Sequences of proteins of immunological interest, NIH Publication No. 91-3242).

In the Fc regions of the PD-1/CD3 bispecific antibody of the present invention, arbitrary amino acids therein may be substituted such that two different heavy chains are easily associated with each other. Preferable examples of embodiments thereof include a PD-1/CD3 bispecific antibody of which in the constant region of the heavy chain having the VH of the first arm specifically binding to PD-1, leucine at position 351 according to the EU numbering system was substituted with lysine, and threonine at position 366 was substituted with lysine, and of which in the constant region of the heavy chain having the VH of the second arm specifically binding to CD3, leucine at position 351 was substituted with aspartic acid, and leucine at position 368 was substituted with glutamic acid. Further, examples thereof also include a PD-1/CD3 bispecific antibody of which in the constant region in the heavy chain having the VH of the first arm specifically binding to PD-1, leucine at position 351 according to the EU numbering system was substituted with aspartic acid, and leucine at position 368 was substituted with glutamic acid, and of which in the constant region in the heavy chain having the VH of the second arm specifically binding to CD3, leucine at position 351 was substituted with lysine, and threonine at position 366 was substituted with lysine.

### The first arm specifically binding to PD-1

In the present specification, the "first arm specifically binding to PD-1" (hereinafter, may be abbreviated as "the first arm") means a part of antibody containing at least a VH of an antibody specifically binding to PD-1 (hereinafter, may be abbreviated as an "anti-PD-1 antibody"), capable of specifically binding to PD-1, regardless of whether it is contained in a part of the antibody or antibody fragment thereof, or exists not as a part but as a simple substance. For example, the first arm like this is composed of a VH of the anti-PD-1 antibody and a VL of the common light chain constituting the same anti-PD-1 antibody. Furthermore, the first arm also includes a Fab part of the antibody containing the same VH and VL. Herein, the term "specifically binding to PD-1" is used as a feature of directly binding to PD-1 with higher binding affinity than at least 1 x 10⁻⁵ M, preferably 1 x 10⁻⁷ M, and more preferably 1 x 10⁻⁹ M (dissociation constant (Kd value)), and not substantially binding to any receptor members belonging to a so-called CD28 family receptor, such as at least, CD28, CTLA-4 and ICOS. Furthermore, the "antibody" in the "antibody specifically binding to PD-1" or in the "anti-PD-1 antibody" means a full-length antibody, that is, a full-length antibody consisting of two heavy chains and two light chains linked with disulfide bonds, and preferably a monoclonal antibody thereof.

Herein, examples of the "first arm specifically binding to PD-1" include one having the VH having
(a) the VH-CDR1 comprising the amino acid sequence represented by HYJ¹LH [wherein J¹ represents G (glycine) or A (alanine), and an alphabet represented by J¹ and other alphabets represent one-letter amino acid abbreviations, respectively],
(b) the VH-CDR2 comprising the amino acid sequence represented by WJ²NTNTU²NPTX²AQGFTG [wherein J² represents L (leucine) or I (isoleucine), U² represents E (glutamic acid) or G (glycine), X² represents F (phenylalanine) or Y (tyrosine), and an alphabet represented by J², U² or X², and other alphabets represent the same as the above, respectively], and
(c) the VH-CDR3 comprising the amino acid sequence represented by GDJ³VVPTTIWNYYU³X³MZ³V [wherein J³ represents M (methionine) or L (leucine), U³ represents H (histidine) or Y (tyrosine), X³ represents F (phenylalanine) or Y (tyrosine), Z³ represents D (aspartic acid) or E (glutamic acid), and an alphabet represented by J³, U³, X³ or Z³, and other alphabets represent the same as the above, respectively].

Herein, preferable examples of embodiments of the "first arm specifically binding to PD-1" include one having
(1a) a VH having the respective VH-CDRs in which J¹ in the HYJ¹LH sequence as VH-CDR1 represents G (glycine), in the WJ²NTNTU²NPTX²AQGFTG as VH-CDR2, J² represents L (leucine), U² represents E (glutamic acid) and X² represents F (phenylalanine), respectively, in the GDJ³VVPTTIWNYYU³X³MZ³V sequence as VH-CDR3, J³ represents M (methionine), U³ represents H (histidine), X³ represents F (phenylalanine) and Z³ represents D (aspartic acid), respectively,
(2a) a VH having the respective VH-CDR in which J¹ in the HYJ¹LH sequence as VH-CDR1 represents G (glycine), in the WJ²NTNTU²NPTX²AQGFTG as VH-CDR2, J² represents I (isoleucine), U² represents G (glycine) and X² represents Y (tyrosine), respectively, in the GDJ³VVPTTIWNYYU³X³MZ³V sequence as VH-CDR3, J³ represents L (leucine), U³ represents H (histidine), X³ represents Y (tyrosine) and Z³ represents E (glutamic acid), respectively,
(3a) a VH having the respective VH-CDR in which J¹ in the HYJ¹LH sequence as VH-CDR1 represents A (alanine), in the WJ²NTNTU²NPTX²AQGFTG as VH-CDR2, J² represents L (leucine), U² represents E (glutamic acid) and X² represents Y (tyrosine), respectively, in the GDJ³VVPTTIWNYYU³X³MZ³V sequence as VH-CDR3, J³ represents M (methionine), U³ represents Y (tyrosine), X³ represents Y (tyrosine) and Z³ represents D (aspartic acid), respectively, or
(4a) a VH having the respective VH-CDR in which J¹ in the HYJ¹LH sequence as VH-CDR1 represents A (alanine), in the WJ²NTNTU²NPTX²AQGFTG as VH-CDR2, J² represents L (leucine), U² represents E (glutamic acid) and X² represents F (phenylalanine), respectively, in the GDJ³VVPTTIWNYYU³X³MZ³V sequence as VH-CDR3, J³ represents M (methionine), U³ represents H (histidine), X³ represents F (phenylalanine) and Z³ represents D (aspartic acid), respectively.

Furthermore, preferable examples of other embodiments of the "first arm specifically binding to PD-1" include one having a VH selected from
(1b) a VH having the VH-CDR1 comprising the amino acid sequence set forth in SEQ ID No. 6, the VH-CDR2 comprising the amino acid sequence set forth in SEQ ID No. 7, and the VH-CDR3 comprising the amino acid sequence set forth in SEQ ID No. 8,
(2b) a VH having the VH-CDR1 comprising the amino acid sequence set forth in SEQ ID No. 9, the VH-CDR2 comprising the amino acid sequence set forth in SEQ ID No. 10, and the VH-CDR3 comprising the amino acid sequence set forth in SEQ ID No. 11,
(3b) a VH having the VH-CDR1 comprising the amino acid sequence set forth in SEQ ID No. 12, the VH-CDR2 comprising the amino acid sequence set forth in SEQ ID No. 13, and the VH-CDR3 comprising the amino acid sequence set forth in SEQ ID No. 14,
(4b) a VH having the VH-CDR1 comprising the amino acid sequence set forth in SEQ ID No. 15, the VH-CDR2 comprising the amino acid sequence set forth in SEQ ID No. 16, and the VH-CDR3 comprising the amino acid sequence set forth in SEQ ID No. 17, and
(5b) a VH having the VH-CDR1 comprising the amino acid sequence set forth in SEQ ID No. 18, the VH-CDR2 comprising the amino acid sequence set forth in SEQ ID No. 19, and the VH-CDR3 comprising the amino acid sequence set forth in SEQ ID No. 20.

Furthermore, the "first arm specifically binding to PD-1" of the present invention also includes those of which one to five arbitrary amino acid residues are substituted with other amino acids (preferably, conservative amino acids thereof) in the respective VH-CDRs of any one of VH selected from the above-mentioned (1a) to (4a) or (1b) to (5b), and which have substantially the same binding activity to PD-1 as that of the original first arm without any substitutions with the same amino acids. Example thereof include those of which one amino acid residue in the VH-CDR1 is substituted with other amino acids (preferably, a conservative amino acid thereof), and one to five arbitrary amino acid residues in the VH-CDR2 or VH-CDR3 are substituted with other amino acids (preferably, conservative amino acids thereof), respectively. As shown in Figure 4, in the respective CDRs of the anti-PD-1 antibody clone corresponding to the first arm specifically binding to PD-1, respectively, amino acids different among the clones or any combination of a plurality thereof can be exchangeable among the clones. Herein, the substitution with a conservative amino acid means the exchangeability with a residue having a similar side-chain. For example, a group of amino acids having an aliphatic side-chain includes glycine, alanine, valine, leucine and isoleucine, a group of amino acids having an aliphatic hydroxyl side-chain includes serine and threonine, a group of amino acids having amide-containing side-chain includes asparagine and glutamine, a group of amino acids having an aromatic side-chain includes phenylalanine, tyrosine and tryptophane, a group of amino acids having a basic side-chain includes lysine, arginine and histidine, and a group of amino acids having a sulfur-containing side-chain includes cysteine and methionine. Examples of preferable substitutions with a conservative amino acid include that among valine, leucine and isoleucine, that between phenylalanine and tyrosine, that between lysine and arginine, that between alanine and valine, as well as that between asparagine and glutamine. Furthermore, herein, the sentence "which have substantially the same binding activity to PD-1 as that of the original first arm without any substitutions with the same amino acids" mentioned above means that the binding activity to PD-1 of the first arm substituted with the amino acids is 95% or more, preferably 98% or more, and more preferably 99% or more to that of the original first arm without any substitutions with the same amino acids.

Furthermore, the "first arm specifically binding to PD-1" in the present invention also includes one having a VH which contains the respective VH-CDRs having the above-mentioned specific amino acid sequence therein, and of which the amino acid sequence of framework region is encoded by a specific germ-line gene or a gene thereof with somatic mutation(s). For example, the VH presented by any one selected from the above-mentioned (1a) to (4a) or (1b) to (5b) can be encoded by the VDJ recombinant gene or gene thereof with somatic mutation(s) in which the germ-line V gene is IGHV7-4-1 and the germ-line J gene is JH6c. Herein, the amino acid sequence encoded by the germ-line V gene IGHV7-4-1 corresponds to that set forth in SEQ ID No. 21 (Figure 3).

The framework regions in the VH of the first arm specifically binding to PD-1 of the present invention may be encoded by the germ-line VDJ recombinant gene with somatic mutation(s). For example, since the FR1, FR2 and FR3 in the VH presented by any one selected from the above-mentioned (1a) to (4a) or (1b) to (5b) of which the germ-line V gene is IGHV7-4-1 are different from an amino acid sequence encoded by the IGHV7-4-1 gene at the positions of the amino acids shown in Figure 4, they have undergone somatic mutations at the respective same positions. For example, as to the FR1, in the amino acid sequence set forth in SEQ ID No. 21, lysine at position 13 may be substituted with glutamine, alanine at position 16 may be substituted with valine or lysine at position 19 may be substituted with methionine, respectively, or which may be substituted in an arbitrary combination of a plurality thereof. As to the FR2, valine at position 37 in the amino acid sequence set forth in SEQ ID No. 21 may be substituted with leucine. As to the FR3, in the amino acid sequence set forth in SEQ ID No. 21, serine at position 77 may be substituted with threonine or cysteine at position 84 may be substituted with serine or asparagine, respectively, or which may be substituted in an arbitrary combination of a plurality thereof. Furthermore, as to the FR4 of the VH presented by any one selected from the above-mentioned (1a) to (4a) or (1b) to (5b), in the amino acid sequence (Trp-Gly-Lys-Gly-Thr-Thr^{∗}-Val-Thr-Val-Ser-Ser) (SEQ ID No. 41) of the FR4 derived from the J gene JH6c, lysine (Lys) may be substituted with glutamine or asparagine, and/or threonine (Thr) marked with an asterisk may be substituted with leucine. The respective FR1, FR2, FR3 and FR4 including combination of any of amino acid substitutions mentioned above have no substantial effect on the functions of the first arm specifically binding to PD-1, and can be used as framework regions.

Further, the "first arm specifically binding to PD-1" of the present invention also includes one which have the respective CDRs having the amino acid sequence specified as the above, and of which the FR amino acid sequences in the VH are encoded by the specific germ-line gene or gene thereof with somatic mutation(s). Examples of such first arms include those having the VH comprising the amino acid sequence set forth in any one selected from SEQ ID Nos. 1 to 5.

Furthermore, examples of such "first arms specifically binding to PD-1" also include those which have a VH comprising an amino acid sequence having at least 80% identity, preferably at least 90% identity, more preferably at least 95% identity, furthermore preferably at least 98% identity, and further more preferably at least 99% identity to the amino acid sequence set forth in any one selected from SEQ ID Nos. 1 to 5, and in which the difference from the VH amino acid sequence of the original first arm has no substantial effect on the binding activity to PD-1 (hereinafter, may be abbreviated as a "homologous first arm"). Herein, the term "% identity" used in comparison of the identity of amino acid sequences is defined as the percentage of amino acid sequence identical to the reference amino acid sequence (herein, when being needed to maximize the sequence identity, the reference amino acid sequence in which the gap has been introduced) when two sequences are aligned. Herein, the sentence "the different from the VH amino acid sequence of the original first arm has no substantial effect on the binding activity to PD-1" means that the binding activity to PD-1 of the homologous first arm is 95% or more, preferably 98% or more, and more preferably 99% or more to the binding activity of the original first arm.

In a yet another embodiment, the "first arm specifically binding to PD-1" of the present invention also includes one having a variable region (herein, the variable region contains a VH and VL constituting it.) of the anti-PD-1 antibody cross-competing for (1) the binding to PD-1 with the first arm having the VH presented by any one selected from the above-mentioned (1a) to (4a) or (1b) to (5b) or the VH comprising the amino acid sequence set forth in any one selected from SEQ ID Nos. 1 to 5 and the VL of common light chain, or (2) the binding to PD-1 with the variable region of the monoclonal antibody specifically binding to PD-1 having the same VH and the VL, and further includes one having the variable region of the anti-PD-1 antibody with which the binding to PD-1 is cross-competed by (3) the first arm having the VH presented by any one selected from the above-mentioned (1a) to (4a) or (1b) to (5b) or the VH comprising the amino acid sequence set forth in any one selected from SEQ ID Nos. 1 to 5 and the VL of common light chain or (4) the variable region of the monoclonal antibody specifically binding to PD-1 having the same VH and the VL. Herein, the sentence "cross-competing for the binding to PD-1" means inhibiting the binding of the first arm to PD-1, regardless of the degree thereof, by binding to the epitope which is the same as or partially overlaps with that of the first arm exemplified in this specification, or that the binding to PD-1 of the antibody binding to the epitope which is the same as or partially overlaps with that of the exemplified first arm is inhibited by the exemplified first arm, regardless of the degree thereof. Whether it cross-competes or not can be evaluated by a competitive binding assay. For example, it can be determined using Biacore analysis, ELISA assay, flow cytometry, enzyme linked immunosorbent assay (ELISA), fluorescence energy transfer method (FRET) or fluorometric microvolume assay technology (FMAT (registered trademark)).

Examples of the first arm cross-competing for the binding to PD-1 by the first arm having the VH presented by the above-mentioned (5b) and the VL of common light chain include the first arm having the VH presented by any one selected from the above-mentioned (1b) to (4b) and the VL of common light chain (preferably, the VL having the VL-CDR1 comprising the amino acid sequence set forth in SEQ ID No. 26, the VL-CDR2 comprising the amino acid sequence set forth in SEQ ID No. 27 and the VL-CDR3 comprising the amino acid sequence set forth in SEQ ID No. 28), and further the first arm having the VH comprising the amino acid sequence set forth in any one selected from SEQ ID Nos. 1 to 4 and the VL of common light chain (preferably, the VL comprising the amino acid sequence set forth in SEQ ID No. 25).

Furthermore, examples of the first arm cross-competing for binding to PD-1 with the first arm having the VH presented by any one selected from the above-mentioned (1b) to (4b) or the VH comprising the amino acid sequence set forth in any one selected from SEQ ID Nos. 1 to 4 and the VL of common light chain include the first arm having the VH presented by the above-mentioned (5b) and the VL of common light chain (preferably, the VL having the VL-CDR1 comprising the amino acid sequence set forth in SEQ ID No. 26, the VL-CDR2 comprising the amino acid sequence set forth in SEQ ID No. 27 and the VL-CDR3 comprising the amino acid sequence set forth in SEQ ID No. 28), and further the first arm having the VH comprising the amino acid sequence set forth in SEQ ID No. 5 and the VL of common light chain (preferably, the VL comprising the amino acid sequence set forth in SEQ ID No. 25).

Herein, preferable examples of the "first arm specifically binding to PD-1" of the present invention include the first arm having the VH presented by any one selected from the above-mentioned (1b) to (5b). Furthermore, as mentioned above, preferable examples of the first arm also include those in which one to five arbitrary amino acid residues are substituted with other amino acids (preferably, conservative amino acids thereof) in the CDRs of the same VH and the same substitutions do not substantially affect the binding activity to PD-1. Furthermore, as mentioned above, they also include those having the VH in which the amino acid sequences of framework regions are encoded by the germ-line V gene IGHV7-4-1 or J gene JH6c or genes thereof with somatic mutation(s). Then, more preferable examples of the first arm include those having the VH comprising the amino acid sequence set forth in any one selected from SEQ ID Nos. 1 to 5.

Furthermore, the first arm specifically binding to PD-1 of the present invention is preferably one having the VL of common light chain, and such a common light chain is preferably the IGVK1-39/JK1 common light chain, more preferably the light chain containing a VL having the CDR1 comprising the amino acid sequence set forth in SEQ ID No. 26, the CDR2 comprising the amino acid sequence set forth in SEQ ID No. 27 and the CDR3 comprising the amino acid sequence set forth in SEQ ID No. 28, and furthermore preferably the light chain containing the VL comprising the amino acid sequence set forth in SEQ ID No. 25. Furthermore, preferable examples of the constant regions of common light chain include the light chain constant region comprising the amino acid sequence set forth in SEQ ID No. 29.

Furthermore, the "first arm specifically binding to PD-1" is preferably one allowing the interaction between PD-1 and PD-L1, interaction between PD-1 and PD-L2 or both of interactions thereof. Herein, the sentence "allowing the interaction between PD-1 and PD-L1, theinteraction between PD-1 and PD-L2 or both of interactions thereof' means that even when there is the PD-1/CD3 bispecific antibody of the present invention at 20-fold excess over the concentration of the soluble form of PD-L1 or PD-L2, the interaction between PD-L1 and PD-1, interaction between PD-L2 and PD-1 or both of interactions thereof is maintained 50% or more, preferably 70% or more and more preferably 80% or more, compared with those when there is no PD-1/CD3 bispecific antibody of the present invention. Furthermore, the definition of "allowing the interaction between PD-1 and PD-L1, interaction between PD-1 and PD-L2 or both of interactions thereof' may have the same meaning as that of "which does not substantially inhibit the interaction between PD-1 and PD-L1, interaction between PD-1 and PD-L2 or both of interactions thereof."

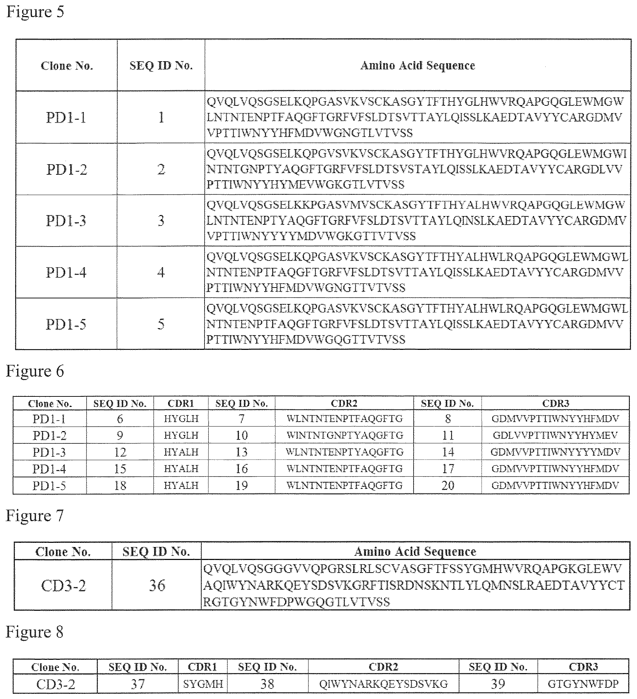

The correspondence relations between the respective anti-PD-1 monoclonal antibody clones obtained to construct the PD-1/CD3 bispecific antibody of the present invention and VH amino acid sequence thereof and SEQ ID numbers thereof are shown in Figure 5. The correspondence relations between the CDR amino acid sequence in the VH of the respective anti-PD-1 monoclonal antibody clones and SEQ ID number thereof are shown in Figure 6.

### The second arm specifically binding to CD3

In the present specification, the "second arm specifically binding to CD3" (hereinafter, may be abbreviated as the "second arm") means an antibody portion having at least a VH of an antibody specifically binding to CD3 (hereinafter, may be abbreviated as an "anti-CD3 antibody") and capable of specifically binding to CD3, regardless of whether it is contained in a part of antibody or antibody fragment, or exists not as a part but as a simple substance. For example, such a second arm is composed of a VH of an anti-CD3 antibody and the VL of common light chain constituting the same anti-CD3 antibody, and further includes a Fab part of antibody containing the same VH and VL. Herein, the sentence "specifically binding to CD3" is used as a feature of directly binding to CD3 with higher binding affinity than at least 1 x 10⁻⁵ M, preferably 1 x 10⁻⁷ M, and more preferably 1 x 10⁻⁹ M (dissociation constant (Kd value)) and not substantially binding to any other proteins. Furthermore, the "antibody" in the "antibody specifically binding to CD3" or "anti-CD3 antibody" means a full-length antibody, that is, a full-length antibody consisting of two heavy chains and two light chains linked with disulfide bonds, and preferably a monoclonal antibody thereof.

Herein, examples of the "second arm specifically binding to CD3" include those having the VH having (1c) the VH-CDR1 comprising the amino acid sequence set forth in SEQ ID No. 37, the VH-CDR2 comprising the amino acid sequence set forth in SEQ ID No. 38 and the VH-CDR3 comprising the amino acid sequence set forth in SEQ ID No. 39.

Furthermore, the "second arm specifically binding to CD3" of the present invention also includes those in which one to five arbitrary amino acid residues are substituted with other amino acids (preferably, conservative amino acids thereof) in the respective CDRs of the VH of the above-mentioned (1c), and which have substantially the same binding activity to CD3 as that of the original second arm without any substitutions with the same amino acids. Examples thereof include one of which one amino acid residue in CDR1 is substituted with other amino acids (preferably, a conservative amino acid thereof), and one to five amino acid residues in CDR2 or CDR3 are substituted with other amino acids (preferably, conservative amino acids thereof), respectively. Herein, the sentence "which have substantially the same binding activity to CD3 as that of the original second arm without any substitutions with the same amino acids" mentioned above means that the binding activity to CD3 of the second arm substituted with the amino acid is 95% or more, preferably 98% or more and more preferably 99% or more to that of the original second arm without any substitutions with the same amino acids. Note here that in examples of the "substitution with conservative amino acids" in the respective VH-CDRs of the second arm include those of the amino acid substitution in the first arm mentioned above.

Furthermore, the "second arm specifically binding to CD3" in the present invention also includes one having a VH which contains the respective CDRs comprising the above-mentioned specific amino acid sequence, and of which the FR amino acid sequences are encoded by a specific germ-line gene or gene thereof with somatic mutation(s). For example, the VH of the above-mentioned (1c) is encoded by a VDJ recombinant gene or gene thereof with somatic mutation(s) in which the germ-line V gene is IGHV3-33. Herein, the amino acid sequence encoded by the V gene IGHV3-33 of the germ line (SEQ ID No. 22) is shown in Figure 3. Further, examples of such second arms include those having the VH comprising the amino acid sequence set forth in SEQ ID No. 36. Furthermore, examples of such second arms also include a VH which comprises an amino acid sequence having at least 80%, preferably at least 90%, more preferably at least 95%, further more preferably at least 98%, and still further preferably at least 99% identity to the amino acid sequence set forth in SEQ ID No. 36, and in which the difference from the VH amino acid sequence of the original second arm has no substantial effect on the binding activity to CD3 (hereinafter, may be abbreviated as a "homologous second arm"). Herein, the sentence "the difference from the VH amino acid sequence of the original second arm has no substantial effect on the binding activity to CD3" means that the binding activity of the homologous second arm to CD3 is 95% or more, preferably 98% or more and more preferably 99% or more to that of the original second arm.

In a yet another embodiment, the "second arm specifically binding to CD3" of the present invention also includes one having a variable region of the anti-CD3 antibody (herein, the variable region includes the VH and VL constituting the variable region) cross-competing for (1) the binding to CD3 with the second arm having the VH presented by the above-mentioned (1c) or the VH comprising the amino acid sequence set forth in SEQ ID No. 36 and the VL of common light chain, or (2) the binding to CD3 with the variable region of the monoclonal antibody specifically binding to CD3 having the same VH and VL. Herein, the "cross-competing for the binding to CD3" means inhibiting the binding of the second arm to CD3, regardless of the degree thereof, by binding to an epitope which is the same as or partially overlaps with the second arm exemplified in the present specification. Herein, whether it cross-competes or not can be similarly evaluated according to the same method as described in descriptions regarding the "first arm specifically binding to PD-1".

Herein, preferable examples of the "second arm specifically binding to CD3" of the present invention include the second arm having the VH presented by the above-mentioned (1c). Furthermore, as mentioned above, these preferable examples of the second arm also include those in which one to five arbitrary amino acid residues are substituted with other amino acids (preferably, conservative amino acids thereof) in the respective CDRs of the VH, and the same substitutions with the amino acid do not substantially affect the binding activity to CD3. Furthermore, as mentioned above, they also include one having the VH in which the amino acid sequences of framework regions are encoded by the germ-line gene IGHV3-33 or gene thereof with somatic mutation(s). Then, more preferable examples of the second arm include those having the VH comprising the amino acid sequence set forth in SEQ ID No. 36.

Hereinafter, the correspondence relations between the respective anti-CD3 antibody clones to construct the PD-1/CD3 bispecific antibody of the present invention, VH amino acid sequence thereof and SEQ ID numbers thereof are shown in Figure 7. The correspondence relations between the respective CDR amino acid sequences in the VH of the respective anti-CD3 antibody clones and SEQ ID numbers thereof are shown in Figure 8.

The second arm specifically binding to CD3 of the present invention is preferably one having a VL of common light chain, and a preferable example of such a common light chain is the IGVK1-39/JK1 common light chain. A more preferable example is the light chain having the VL having the CDR1 comprising the amino acid sequence set forth in SEQ ID No. 26, the CDR2 comprising the amino acid sequence set forth in SEQ ID No. 27 and the CDR3 comprising the amino acid sequence set forth in SEQ ID No. 28, and a further preferable example is the light chain having the VL comprising the amino acid sequence set forth in SEQ ID No. 25. Furthermore, preferable examples of the constant region of the common light chain include the light chain constant region comprising the amino acid sequence set forth in SEQ ID No. 29.

Preferable examples of the "second arm specifically binding to CD3" of the present invention include those specifically binding to CD3ε.

An isotype of the PD-1/CD3 bispecific antibody of the present invention is preferably an IgG antibody, further preferably an IgG₁ antibody or IgG₄ antibody, and more preferably an IgG₁ antibody.

Examples of preferable embodiments of the PD-1/CD3 bispecific antibodies of the present invention include those of which the first arm specifically binding to PD-1 has
(A) the VH in which one to five arbitrary amino acid residues may be substituted with other amino acids (preferably, conservative amino acids thereof) in any one or more of the CDRs selected from the VH-CDR1, VH-CDR2 and VH-CDR3 in the VH presented by any selected from the above-mentioned (1a) to (4a) or (1b) to (5b), and
(B) the VL of common light chain having the CDR1 comprising the amino acid sequence set forth in SEQ ID No. 26, the CDR2 comprising the amino acid sequence set forth in SEQ ID No. 27 and the CDR3 comprising the amino acid sequence set forth in SEQ ID No. 28, and the second arm specifically binding to CD3 has
(C) the VH in which one to five arbitrary amino acid residues may be substituted with other amino acids (preferably, conservative amino acids thereof) in any one or more of the CDRs selected from the VH-CDR1, VH-CDR2 and VH-CDR3 in the VH presented by the above mentioned (1c), and
(D) the VL of common light chain having the CDR1 comprising the amino acid sequence set forth in SEQ ID No. 26, the CDR2 comprising the amino acid sequence set forth in SEQ ID No. 27 and the CDR3 comprising the amino acid sequence set forth in SEQ ID No. 28.

Examples of more preferable embodiments thereof include the PD-1/CD3 bispecific antibody of which the first arm specifically binding to PD-1 has
(A) the VH presented by any one selected from the above (1a) to (4a) or (1b) to (5b), and
(B) the VL of common light chain having the CDR1 comprising the amino acid sequence set forth in SEQ ID No. 26, the CDR2 comprising the amino acid sequence set forth in SEQ ID No. 27 and the CDR3 comprising the amino acid sequence set forth in SEQ ID No. 28, and the second arm specifically binding to CD3 has
(C) the VH presented by the above (1c), and
(D) the VL of common light chain having the CDR1 comprising the amino acid sequence set forth in SEQ ID No. 26, the CDR2 comprising the amino acid sequence set forth in SEQ ID No. 27 and the CDR3 comprising the amino acid sequence set forth in SEQ ID No. 28.

Furthermore, examples of other preferable embodiments of the PD-1/CD3 bispecific antibody of the present invention include those of which the first arm specifically binding to PD-1 has
(A) the VH comprising the amino acid sequence set forth in any one selected from SEQ ID Nos. 1 to 5, or a VH comprising an amino acid sequence having at least 80% identity to VH amino acid sequence thereof, and
(B) the VL of common light chain comprising the amino acid sequence set forth in SEQ ID No. 25, and
the second arm specifically binding to CD3 has
(C) the VH comprising the amino acid sequence set forth in SEQ ID No. 36, or a VH comprising an amino acid sequence having an identity of at least 80% to VH amino acid sequence thereof, and
(D) the VL of common light chain comprising the amino acid sequence set forth in SEQ ID No. 25.

Examples of other more preferable embodiments thereof include the PD-1/CD3 bispecific antibody of which the first arm specifically binding to PD-1 has
(A) the VH comprising the amino acid sequence set forth in any one selected from SEQ ID Nos. 1 to 5, and
(B) the VL of common light chain comprising the amino acid sequence set forth in SEQ ID No. 25, and
the second arm specifically binding to CD3 has
(C) the VH comprising the amino acid sequence set forth in SEQ ID No. 36, and
(D) the VL of common light chain comprising the amino acid sequence set forth in SEQ ID No. 25.

In the PD-1/CD3 bispecific antibody of the present invention, when it is an IgG₁ antibody, the IgG₁ antibody in which each leucine at position 235 according to the EU numbering system was substituted with glycine, and/or each glycine at position 236 was substituted with arginine on two heavy chain constant regions or a hinge region thereof is preferable. Furthermore, a bispecific antibody in which the C-terminal amino acids of heavy chains, for example, each lysine at position 447 according to the EU numbering system have been deleted is preferable. Furthermore, when the PD-1/CD3 bispecific antibody is an IgG₄ antibody, an antibody in which serine at position 228 according to the EU numbering system, located in the hinge region, was substituted with proline is preferable.

Furthermore, when the PD-1/CD3 bispecific antibody is an IgG₁ antibody, preferable embodiments thereof includes those in which in the constant region of the heavy chain having the VH of the first arm specifically binding to PD-1, leucine at position 351 according to the EU numbering system was substituted with lysine and threonine at position 366 was substituted with lysine, and in the constant region of the heavy chain having the VH of the second arm specifically binding to CD3, leucine at position 351 was substituted with aspartic acid and leucine at position 368 was substituted with glutamic acid. Furthermore, an IgG₁ antibody in which in the constant region of the heavy chain having the VH of the first arm specifically binding to PD-1, leucine at position 351 according to the EU numbering system was substituted with aspartic acid and leucine at position 368 was substituted with glutamic acid, and in the constant region of the heavy chain having the VH of the second arm specifically binding to CD3, leucine at position 351 was substituted with lysine and threonine at position 366 was substituted with lysine is also preferable, as well.

Furthermore, preferable embodiments, when the PD-1/CD3 bispecific antibody is an IgG₁ antibody, include the IgG₁ antibody of which in two heavy chain constant regions, (1) each methionine at position 252 according to the EU numbering system was substituted with glutamic acid, proline, arginine or aspartic acid, (2) each asparagine at position 434 according to the EU numbering system was substituted with leucine, and/or (3) each glutamine at position 438 according to the EU numbering system was substituted with glutamic acid. It is expected that by these amino acid substitutions, the half-life in blood of the PD-1/CD3 bispecific antibody can be shorted at least 50%, preferably at least 60%, more preferably at least 70%, furthermore preferably at least 80%, and most preferably at least 90%, compared to those without the same amino acid substitutions.

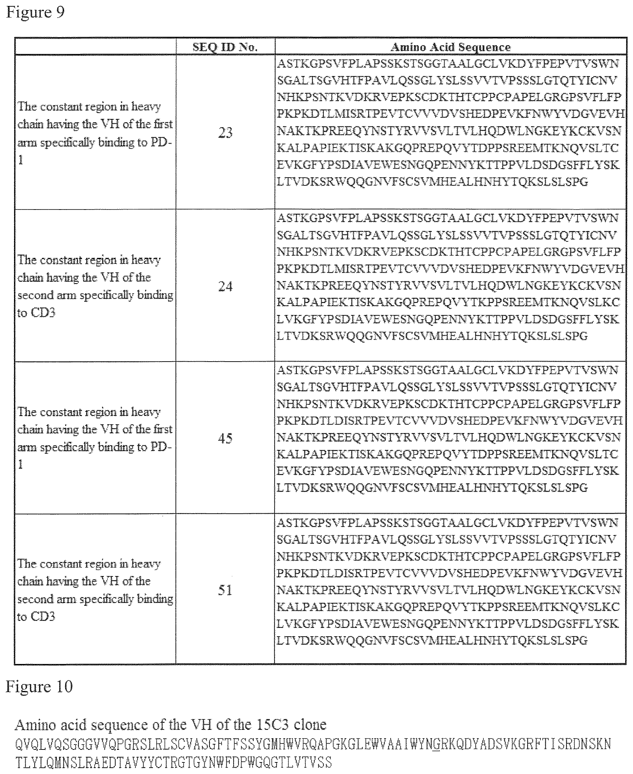

Examples of preferable embodiments of the PD-1/CD3 bispecific IgG₁ antibody in which the above-mentioned amino acid substitutions in the heavy chain constant region were taken include those in which the heavy chain having the VH of the first arm specifically binding to PD-1 has the heavy chain constant region comprising the amino acid sequence set forth in any one selected from SEQ ID No. 23, SEQ ID No. 42, SEQ ID No. 43, SEQ ID No. 44, SEQ ID No. 45, SEQ ID No. 46 and SEQ ID No. 47, and the heavy chain having the VH of the second arm specifically binding to CD3 has the heavy chain constant region comprising the amino acid sequence set forth in any one selected from SEQ ID No. 24, SEQ ID No. 48, SEQ ID No. 48, SEQ ID No. 50, SEQ ID No. 51, SEQ ID No. 52 and SEQ ID No. 53. Some of those amino acid sequences are shown in Figure 9.

The most embodiments of the PD-1/CD3 bispecific antibody of the present invention are preferably the clones PD1-1(Bi), PD1-2(Bi), PD1-3(Bi), PD1-4(Bi) and PD1-5(Bi) generated in Example 8 of the present specification, and the clones Mut1, Mut2, Mut3, Mut4, Mut5 and Mut6 clone generated in Example 19.

Examples of preferable features of the PD-1/CD3 bispecific antibody of the present invention include (1) allowing the interaction between PD-1 and PD-L1, interaction between PD-1 and PD-L2 or both of interactions thereof and/or (2) sufficiently reducing cytokine production. Herein, the sentence "allowing the interaction between PD-1 and PD-L1, interaction between PD-1 and PD-L2 or both interactions" means the same as described in descriptions regarding the "first arm specifically binding to PD-1." On the other hand, the sentence "sufficiently reducing cytokine production" means that, for example, during intravenous administration or by 24 hours after that administration, by drip infusion of the PD-1/CD3 bispecific antibody of the present invention, for example, concentration in blood or tissue of cytokine including IL-2, IFN-γ and/or TNF-α in blood or tissue is not increased or even if it increases, it is such a degree that it can be suppressed by steroid administration.

### Method for manufacturing and purifying the PD-1/CD3 bispecific antibody

The PD-1/CD3 bispecific antibody and antibody fragment thereof of the present invention can also be manufactured by the method disclosed in WO2014/051433, WO2013/157953 or WO2013/157954.

Specifically, it can be manufactured by gene-transferring an expression vector in which (1) a polynucleotide encoding a heavy chain having the VH of the first arm specifically binding to PD-1, (2) a polynucleotide encoding a heavy chain having the VH of the second arm specifically binding to CD3, and (3) a polynucleotide encoding a common light chain have been inserted, respectively, into mammalian animal cells to transform them, and then by having them express and secret both of the heavy chain and common light chain.

Herein, any host cells for expressing the PD-1/CD3 bispecific antibody of the present invention can be used as long as they can be gene-introduced by expression vectors to express them. Preferable examples of host cells include insect cells such as SF-9 and SF-21, more preferably, mammalian cells such as mouse cells including CHO cells, BHK cells, SP2/0 cells and NS-0 myeloma cells, primate cells such as COS and Vero cells and MDCK cells, BRL 3A cells, hybridoma, tumor cells, immortalized primary cells, W138, HepG2, HeLa, HEK293, HT1080 and embryonic retina cells such as PER.C6, and the like. Note here that in selection of the expression system, expression vectors for mammalian cells and hosts cells therefor can often be used such that antibodies are appropriately glycosylated. Human cell lines, preferably PER.C6 are advantageously used to obtain antibodies corresponding to glycosylated patterns for human.

Protein production in host cells transformed by gene-transferring the expression vectors can be carried out with reference to, for example, Current Protocols in Protein Science (1995), Coligan JE, Dunn BM, Ploegh HL, Speicher DW, Wingfield PT, ISBN 0-471-11184-8, Bendig, 1988. Furthermore, general guidelines, procedures and practical methods to maximize the productivity of host cell culture can be carried out with reference to Mammalian Cell Biotechnology: a Practical Approach (M. Butler, ed., IRL Press, 1991). Expression of antibodies in host cells is described in, for example, publications such as EP0120694, EP0314161, EP0481790, EP0523949, US4816567, WO2000/63403 and the like.

Herein, culture conditions for host cells can be optimized by well-known methods, and the amount of protein production therein can be optimized. The culture can be carried out by batch culture, feeding culture, continuous culture or hollow-fiber culture in a petri dish, roller bottle or reaction chamber. In order to produce recombinant protein by cell culture in a large scale and continuously, it is preferable to allow cells to proliferate in suspension. Furthermore, it is more preferable to culture cells under a condition without any animal- or human-derived serum or animal- or human-derived serum components.

Antibodies expressed in host cells and recovered from them or culture thereof by well-known methods can be purified using well-known methods. Examples of purification method include immunoprecipitation method, centrifugation method, filtration, size-exclusion chromatography, affinity chromatography, cation and/or anion exchange chromatography, hydrophobic interaction chromatography and the like. Furthermore, protein A or protein G affinity chromatography may be preferably used (see, e.g., US4801687 and US5151504).

### [Pharmaceutical use]

The PD-1/CD3 bispecific antibody of the present invention is useful for preventing, suppressing the progression of symptoms of or the recurrence of and/or treating autoimmune diseases or graft-versus-host diseases (GVHD).

Examples of autoimmune diseases which can be prevented, of which the progression of symptoms can be suppressed and/or which can be treated by the PD-1/CD3 bispecific antibody or the like of the present invention include Behcet's disease, systemic lupus erythematosus, chronic discoid lupus erythematosus, multiple sclerosis, systemic scleroderma, progressive systemic sclerosis, scleroderma, polymyositis, dermatomyositis, periarteritis nodosa (polyarteritis nodosa and microscopic polyangiitis), aortitis syndrome (Takayasu's arteritis), malignant rheumatoid arthritis, rheumatoid arthritis, juvenile idiopathic arthritis, spondylarthritis, mixed connective tissue disease, Castleman's disease, Sjogren's syndrome, adult Still's disease, vasculitis, allergic granulomatous vasculitis, hypersensitivity vasculitis, rheumatoid vasculitis, large vessel vasculitis, ANCA associated vasculitis (e.g., granulomatosis with polyangiitis and eosinophilic granulomatosis with polyangiitis), Cogan's syndrome, RS3PE syndrome, temporal arteritis, polymyalgia rheumatica, fibromyalgia, antiphospholipid antibody syndrome, eosinophilic fasciitis, IgG4-related disease (e.g., primary sclerosing cholangitis and autoimmune insulitis, etc.), Guillain-Barre syndrome, myasthenia gravis, chronic atrophic gastritis, autoimmune hepatitis, non-alcoholic steatohepatitis, primary biliary cirrhosis, Goodpasture's syndrome, rapidly progressive glomerulonephritis, lupus nephritis, megaloblastic anemia, autoimmune hemolytic anemia, pernicious anemia, autoimmune neutropenia, idiopathic thrombocytopenic purpura, Basedow disease (Graves' disease (hyperthyroidism)), Hashimoto disease, autoimmune adrenal insufficiency, primary hypothyroidism, Addison's disease (chronic hypoadrenocorticism), idiopathic Addison's disease, type I diabetes mellitus, slowly progressive type I diabetes mellitus (latent autoimmune diabetes in adult), focal scleroderma, psoriasis, psoriatic arthritis, bullous pemphigoid, pemphigus, pemphigoid, gestational herpes, linear IgA bullous dermatosis, acquired epidermolysis bullosa, alopecia areata, vitiligo, vitiligo vulgaris, neuromyelitis optica, chronic inflammatory demyelinating polyneuropathy, multifocal motor neuropathy, sarcoidosis, giant cell arteritis, amyotrophic lateral sclerosis, Harada disease, autoimmune optic neuropathy, idiopathic azoospermia, habitual abortion, inflammatory bowel disease (e.g., ulcerous colitis and Crohn's disease), celiac disease, ankylosing spondylitis, severe asthma, chronic urticaria, transplantation immunity, familial Mediterranean fever, eosinophilic chronic rhinosinusitis, dilated cardiomyopathy, systemic mastocytosis, inclusion body myositis and the like.

In the present invention, the term "treating" means cure or improvement of a disease or symptom thereof. The term "preventing" means that the onset of a disease or symptom thereof is prevented or delayed for a certain period of time. The term "suppressing of the progression of symptoms" means that the progress or aggravation of symptoms is suppressed to stop the progress of disease conditions. The meaning of "preventing" includes suppressing the recurrence. The term "suppressing the recurrence" means that the recurrence of diseases or syndromes is prevented or a possibility of the recurrence is reduced.

The PD-1/CD3 bispecific antibody or the like of the present invention is usually administered systemically or locally through parenteral administration. Specific examples of such administration methods include injection administration, intranasal administration, transpulmonary administration, percutaneous administration and the like. Examples of injection administration include intravenous injection, intramuscular injection, intraperitoneal injection and the like. For intravenous injection, drip intravenous infusion is preferable. The dose thereof varies depending on the age, body weight, symptoms, therapeutic effect, administration method, treating period and the like. The single dose thereof for an adult patient is usually within a range of 0.1 µg/kg to 300 mg/kg and particularly preferably within a range of 0.1 mg/kg to 10 mg/kg, once to several times per day by parenteral administration, or within a range of 30 minutes to 24 hours per day by intravenous sustaining administration. Needless to say, as mentioned above, since the dose varies depending on various conditions, it may be lower than the above-mentioned dose, or may be needed to be more than the above.

### Formulation

When the PD-1/CD3 bispecific antibody or the like of the present invention is formulated to be used as an injection or infusion solution for drip infusion, the injection or infusion solution may be in any form of an aqueous solution, suspension or emulsion, or may be formulated as a solid agent along with pharmaceutically acceptable carriers such that it can be dissolved, suspended or emulsified by adding a solvent at the time of use. Examples of solvents which can be used in the injection or the infusion solution for drip infusion include distilled water for injection, physiological saline, glucose solutions and isotonic solutions and the like (e.g., solutions in which sodium chloride, potassium chloride, glycerin, mannitol, sorbitol, boric acid, borax, propylene glycol or the like is dissolved.).

Herein, examples of the pharmaceutically acceptable carriers include a stabilizer, solubilizer, suspending agent, emulsifier, soothing agent, buffering agent, preservative, antiseptic agent, pH adjuster, antioxidant and the like. As a stabilizer, for example, various amino acids, albumin, globulin, gelatin, mannitol, glucose, dextran, ethylene glycol, propylene glycol, polyethylene glycol, ascorbic acid, sodium bisulfite, sodium thiosulfate, sodium edetate, sodium citrate, dibutylhydroxytoluene or the like can be used. As a solubilizer, for example, alcohol (e.g., ethanol etc), polyalcohol (e.g., propylene glycol and polyethylene glycol, etc.), nonionic surfactant (e.g., Polysorbate 20 (registered trademark), Polysorbate 80 (registered trademark) and HCO-50, etc.) or the like can be used. As a suspending agent, for example, glyceryl monostearate, aluminum monostearate, methyl cellulose, carboxymethyl cellulose, hydroxymethyl cellulose, sodium lauryl sulfate or the like can be used. As an emulsifier, for example, gum arabic, sodium alginate, tragacanth or the like can be used. As a soothing agent, for example, benzyl alcohol, chlorobutanol, sorbitol or the like can be used. As the buffering agents, for example, phosphate buffer, acetate buffer, borate buffer, carbonate buffer, citrate buffer, Tris buffer, glutamic acid buffer, epsilon aminocaproic acid buffer or the like can be used. As a preservative, for example, methyl parahydroxybenzoate, ethyl parahydroxybenzoate, propyl parahydroxybenzoate, butyl parahydroxybenzoate, chlorobutanol, benzyl alcohol, benzalkonium chloride, sodium dehydroacetate, sodium edeate, boric acid, borax or the like can be used. As an antiseptic agent, for example, benzalkonium chloride, parahydroxybenzoic acid, chlorobutanol or the like can be used. As a pH adjuster, for example, hydrochloric acid, sodium hydroxide, phosphoric acid, acetic acid or the like can be used. As an antioxidant, for example, (1) aqueous antioxidants such as ascorbic acid, cysteine hydrochloride, sodium bisulfate, sodium metabisulfite and sodium sulfite, (2) oil-soluble antioxidants such as ascorbyl palmitate, butylated hydroxy anisole, butylated hydroxy toluene, lecithin, propyl gallate and α-tocopherol, and (3) metal chelating agents such as citric acid, ethylenediaminetetraacetic acid, sorbitol, tartaric acid and phosphoric acid can be used.

The injection or infusion solution for drip infusion can be produced by performing sterilization in the final process, or aseptic manipulation, for example, sterilization by filtration with a filter or the like and subsequently filling it to an aseptic container. The injection or infusion solution for drip infusion may be used by dissolving the vacuum dried or lyophilized aseptic powder (which may include a pharmaceutically acceptable carrier powders) in an appropriate solvent at the time of use.

### Combination use or combination formulation

Furthermore, the PD-1/CD3 bispecific antibody of the present invention may be used in combination with other agents which is used for preventing, suppressing the progression of symptoms of or the recurrence of and/or treating autoimmune disease. In the present invention, examples of administration forms in combinational use with the other agents (combinational use) may include a form of combination formulation containing both of ingredients in one formulation and a form being administered in separate formulations. Such combinational uses can complement the effects on preventing, suppressing the progression of symptoms of, suppressing the recurrence of and/or treating by other agents, or can maintain or reduce the dose or frequency of administration of other agents. When separately administering the PD-1/CD3 bispecific antibody or the like of the present invention and other agents, they may be simultaneously administered for a certain period of time, and then only the PD-1/CD3 bispecific antibody or the like or other agents may be administered. Alternatively, the PD-1/CD3 bispecific antibody or the like of the present invention may be initially administered, and after completion of administration thereof, other agents may be administered. Other agents may be initially administered, and after completion of administration thereof, the PD-1/CD3 bispecific antibody or the like of the present invention may be administered. The respective administration methods may be the same as or different from each other. A kit containing a formulation containing the PD-1/CD3 bispecific antibody of the present invention and a formulation containing other agents can also be provided. Herein, the doses of other agents can be appropriately selected based on the dose in clinical use. Further, other agents may be administered in combination of two or more kinds of arbitrary agents at an appropriate ratio. Furthermore, examples of other agents include not only those already known but also those newly discovered in the future.

For example, when the PD-1/CD3 bispecific antibody or the like of the present invention is applied for preventing, suppressing the progression of symptoms of or the recurrence of and/or treating type I diabetes mellitus, it may be used in combination with any one or more of agents selected from an insulin preparation (e.g., human insulin, insulin glargine, insulin lispro, insulin detemir and insulin aspart, etc.), sulfonylurea agent (e.g.., glibenclamide, gliclazide and glimepiride, etc.), quick-acting insulin secretion promoter (e.g., nateglinide etc.), biguanide preparation (e.g., metformin etc.), insulin resistance improving agent (e.g., pioglitazone etc.), α-glucosidase inhibitor (e.g., acarbose and voglibose, etc.), diabetic neuropathy therapeutic agent (e.g., epalrestat, mexiletine and imidapril, etc.), GLP-1 analog preparation (e.g., liraglutide, exenatide and lixisenatide, etc.), DPP-4 inhibitor (e.g., sitagliptin, vildagliptin and alogliptin, etc.) and the like.

Furthermore, for example, when the PD-1/CD3 bispecific antibody or the like of the present invention is applied for preventing, suppressing the progression of symptoms of or the recurrence of and/or treating multiple sclerosis, it may be used in combination with any one or more of agents selected from a steroid agent (e.g., cortisone, cortisone acetate, hydrocortisone, hydrocortisone sodium phosphate, hydrocortisone sodium succinate, fludrocortisone acetate, prednisolone, prednisolone acetate, prednisolone sodium succinate, prednisolone butylacetate sodium, prednisolone phosphate, halopredone acetate Ester, methylprednisolone, methylprednisolone acetate, methylprednisolone sodium succinate, triamcinolone, triamcinolone diacetate, triamcinolone acetonide, dexamethasone, dexamethasone acetate, dexamethasone valerate, dexamethasone cipecilate, dexamethasone palmitate, dexamethasone propionate, dexamethasone sodium phosphate, dexamethasone sodium metasulfobenzoate, parameterzone, parameterzone acetate, betamethasone, betamethasone dipropionate, betamethasone valerate, betamethasone acetate, betamethasone butyrate propionate and betamethasone sodium phosphate, etc.), interferon β-1a, interferon β-1b, glatiramer acetate, mitoxantrone, azathioprine, cyclophosphamide, cyclosporin, methotrexate, cladribine, adrenocorticotropic hormone (ACTH), corticotropin, mizoribine, tacrolimus, fingolimod, alemtuzumab and the like.

Furthermore, for example, when the PD-1/CD3 bispecific antibody or the like of the present invention is applied for preventing, suppressing the progression of symptoms of or the recurrence of and/or treating systemic lupus erythematosus, it may be used in combination with any one or more of agents selected from a steroid agent (e.g., steroid agents mentioned above), immunosuppressive agent (e.g., cyclosporin, tacrolimus and fingolimod, etc.) and belimumab.

For example, when the PD-1/CD3 bispecific antibody or the like of the present invention is applied for preventing, suppressing the progression of symptoms of or the recurrence of and/or treating rheumatoid arthritis, it may be used in combination with any one or more of agents selected from a steroid agent (e.g., steroid agents mentioned above), anti-rheumatic agent (e.g., methotrexate, sulfasalazine, bucillamine, leflunomide, mizoribine and tacrolimus, etc.), anti-cytokine agent (e.g., infliximab, adalimumab, tocilizumab, etanercept, golimumab and certolizumab, etc.), abatacept and the like.

When being applied for preventing, suppressing the progression of symptoms of or the recurrence of and/or treating other autoimmune diseases, the PD-1/CD3 bispecific antibody or the like of the present invention may be used in combination with any one or more of the above-mentioned other agents.

The present invention will now be described in more detail by the following examples, but the scope of the present invention is not limited thereto. A person skilled in the art can make various changes and modifications, based on the description of the present invention, and such changes and modifications are also included in the present invention.

### [Examples]

### Example 1: Immunization of MeMo (registered trademark) mice using recombinant human PD-1-Fc fusion protein

As a method for obtaining the first arm specifically binding to PD-1 of the present invention, a method for immunizing MeMo (registered trademark) mice (see WO2009/157771) with a recombinant human PD-1 protein was selected. The MeMo (registered trademark) mice are those genetically modified such that a gene fragment containing a non-recombinant human heavy chain V gene region, D gene region and J gene region, as well as the recombinant human κ light chain IgVκ1-39^{∗}01/IGJκ1^{∗}01 germ-line gene have been linked to a mouse constant region gene. By directly immunizing them with a target protein for antibody, antibodies composed of heavy chains and common light chains, with diversity, can be produced.

Twelve 12- to 16-week-old MeMo (registered trademark) MS5B/MS9 mice were immunized with recombinant human PD-1-Fc fusion protein (R&D Systems, serial number 1086-PD) emulsified using Gerbu adjuvant MM (Gerbu Biotechnik, serial number #3001) at an interval of 14 days. On days 0, 14 and 28 after immunization, the recombinant human PD-1-Fc fusion protein was subcutaneously administered, and thereafter, the recombinant human PD-1-Fc fusion protein dissolved in PBS was administered subcutaneously. On days 21, 35, 56, 77 and 98 after immunization, the antibody titer in serum was evaluated by flow cytometry using human PD-1-forced expressed HEK293 T cell lines. When the human PD-1-forced expressed HEK293T cell lines were stained in 1000-fold diluted serum, mouse lymph tissues of which the MFI value increased more than three times higher than that of the human PD-1 non-expressing HEK293T cell lines as a control were used for constructing a phage display library. Mice meeting the criteria for constructing the library were additionally immunized with the recombinant PD-1-Fc fusion protein for three days from the evaluation date of the antibody titer, and of which spleens and inguinal lymph nodes were collected. Spleens and inguinal lymph nodes were also collected from mice in which the antibody titer in serum to human PD-1 and cynomolgus monkey PD-1 was 1/100 or more, and the antibody titer was not increased by additional immunization. RNA was extracted from those lymphoid tissues, and then cDNA synthesis was carried out.

### Example 2: Construction of phage display library to obtain an anti-PD-1 antibody having the first arm specifically binding to PD-1 (protein immunization)

Using the DNA prepared in Example 1 and primers specific to immunoglobulin heavy chain variable region family, PCR reaction was carried out. The obtained PCR products were digested with restriction enzymes SfiI and XhoI, and inserted into MV1473 phagemid vector [having a gene (human κ light chain IgVκ1-39^{∗}01/IGJκ1^{∗}01 germ-line gene) encoding the common light chain] digested with the same restriction enzymes to construct the library.

### Example 3: Screening of an anti-PD-1 antibody having the first arm specifically binding to PD-1

Using plates coated with human PD-1-Fc fusion protein, human PD-1-His tag fusion protein, cynomolgus monkey PD-1-His tag fusion protein or mouse PD-1-His tag fusion protein, phage selection based on the binding property to PD-1 was carried out. When using human PD-1-Fc fusion protein, during incubation with phage, human IgG (SIGMA, serial number 14506) was added thereto to absorb Fc reactive clones. Binding phages capable of binding to human PD-1, cynomolgus monkey PD-1 and mouse PD-1 were enriched. Using selections on cynomolgus monkey PD-1 expressing HEK293 T cell lines, phages capable of binding to cynomolgus monkey PD-1 were enriched. Escherichia coli strain TG1 clones transformed with phages obtained by selection were obtained to produce a master plate.

Further, based on the binding property to PD-1 on a plate to which human PD-1-Fc fusion protein was adsorbed, the phage selection from periplasmic space extracts of the clones obtained by the above-mentioned selection was carried out. Note here that as the criteria for selection, clones with signals three times more than signal (OD₄₅₀ value) in a negative control well (PBS) were defined as positive clones.

### Example 4: DNA sequencing of candidate clones for anti-PD-1 antibodies having the first arm specifically binding to PD-1

DNA sequencing for heavy chain variable region genes of positive clones obtained by screening in Example 3 was carried out. Analyzed DNA sequences were classified into super clusters (a group having the same-length CDR3, in which an amino acid sequence of CDR3 is 70% or more homologous each other) and clusters (a group in which amino acid sequences of the heavy chain CDR3 are same). 924 clones were obtained, which were classified into 146 types super clusters and 194 types clusters.

### Example 5: Screening based on the evaluation of the binding property to PD-1 expressing cells

From the respective classified super clusters, anti-PD-1 monoclonal antibody clones meeting the following conditions were screened and isolated
(1) having somatic mutations in CDRs with high frequency,
(2) having a germline gene of highly frequently used VH, and
(3) having a high signal in the screening based on the binding property to human PD-1-Fc fusion protein.

Using Fab fragments contained in those periplasmic space extracts, the binding properties to human PD-1 expressing CHO-S cell lines and cynomolgus monkey PD-1 expressing CHO-S cell lines were evaluated by detecting with anti-mouse IgG polyclonal antibodies. Among the evaluated 117 clones (105 types of clusters), in 22 clones including the anti-PD-1 monoclonal antibody clones PD1-1, PD1-2, PD1-3 and PD1-4, the bindings to the human PD-1 expressing CHO-S cell lines were detected.

### Example 6: Preparation of amino acid substituted products of the anti-PD-1 monoclonal antibody having the first arm specifically binding to PD-1

The clones PD1-1 and PD1-4 contain deamidation motifs (Asn-Gly) in the framework region 4 of heavy chain variable region thereof, respectively. In order to obtain the first arm with reduced risk of deamidation, a variant in which the deamidation motifs have been converted was prepared. Asparagine (Asn) at position 119 according to the EU numbering system for the clone PD1-4 was altered to glutamine by a well-known site-specific mutation method, to prepare and isolate the clone PD1-5. The binding property to human PD-1 expressing CHO-S cells of the same clone was equal to that of the clone PD1-4.

### Example 7: Screening of anti-CD3 monoclonal antibodies having the second arm specifically binding to CD3

In order to obtain Fabs binding to CD3 having more stability and further reduced charge heterogeneity, using the anti-CD3 antibody clone having the VH of the anti-CD3 antibody 15C3 clone described in WO2005/118635 and IGVK1-39/JK1 common light chain, an anti-CD3 antibody having the "second arm specifically binding to CD3" of the present invention was obtained by the following method.

By converting the underlined 55th glycine in the VH amino acid sequence of 15C3 shown in Figure 10, into alanine, the anti-CD3 antibody clone CD3-1, having the binding property to human CD3 equal to the above-mentioned clone and having improved charge heterogeneity was obtained.

Furthermore, in order to obtain a plurality of Fabs binding to CD3, capable of improving the VH/VL interaction with the common light chain (IgVκl-39^{∗}01/IGJκ1^{∗}01), a phage display library expressing a plurality of Fabs consisted of VH variants of the CD3-1 clone in which that amino acid residues thereof have been substituted was constructed, based on the VH of CD3-1 clone. This phage library was screened using HBP-ALL cells or recombinant human CD3ε-Fc protein. Phages binding to recombinant human CD3ε-Fc protein were chemically eluted, and used for reinfection to bacteria. A plurality of survived bacterial colonies were extracted, then phages therefrom were extracted, and then screened by flow cytometry, based on the binding to CD3 expressed on cell surface. All of the phages binding to CD3 were subjected to colony PCR to amplify cDNAs encoding VHs thereof, and DNA sequences thereof were determined, respectively.

As a result, the obtained anti-CD3 antibody clone CD3-2 having the VH comprising the amino acid sequence set forth in SEQ ID No. 36 showed the binding property to CD3 and charge homogeneity equivalent to those of the clone CD3-1.

### Example 8: Preparation of the PD-1/CD3 bispecific monoclonal antibody

Expression vectors expressing the respective heavy chains of the first arm specifically binding to PD-1 were prepared by linking DNAs encoding heavy chain variable regions of the anti-PD-1 monoclonal antibody clones PD1-1 to PD1-5 and PD1-6 selected in Example 5, respectively, to DNAs encoding IgG₁ heavy chain constant regions, respectively. On the other hand, an expression vector expressing the heavy chain of the second arm specifically binding to CD3 was prepared by linking a DNA encoding the heavy chain variable region of the anti-CD3 monoclonal antibody clone CD3-2 selected in Example 7 to a DNA encoding an IgG₁ heavy chain constant region. Herein, as genes expressing the heavy chain constant region, as to the first arm specifically binding to PD-1, a gene expressing Fc region having L351D/L368E variation (DE variation) was used. As to the second arm specifically binding to CD3, a gene expressing Fc region having L351K/T366K variation (KK variation) was used. These expression vectors were constructed so as to further contain a gene encoding the IGVK1-39/JK1 common light chain such that it is expressed together. Further, in order to eliminate the Fc effector activity, the genes expressing these heavy chain constant regions have been modified so as to be expressed as those in which each leucine at position 235 was substituted with glycine and further each glycine at position 236 was substituted with arginine in the heavy chain constant regions, and furthermore in order to avoid processing after translation, those modified so as to be expressed as those in which each lysine at a position 447 of the C-terminus of the heavy chain constant region was deleted were used. Both of these expression vectors were gene-transferred into Free Style 293F cells to make them produce antibodies in culture supernatants. The culture supernatants were collected and then treated by protein A affinity chromatography, to purify the clones PD1-1(Bi), PD1-2(Bi), PD1-3(Bi), PD1-4(Bi) and PD1-5(Bi) as the PD-1/CD3 bispecific monoclonal antibody of the present invention, respectively. Furthermore, the clone PD1-6(Bi) was also produced by the same method. Note here that, in that having the first arms specifically binding to PD-1 derived from the anti-PD-1 monoclonal antibody clones PD1-1, PD1-2, PD1-3, PD1-4 and PD1-5, as well as the clone PD1-6, used in production thereof, these the PD-1/CD3 bispecific monoclonal antibodies correspond to those anti-PD-1 monoclonal antibody clones, respectively.

### Example 9: Evaluation of the binding property of the PD-1/CD3 bispecific monoclonal antibody

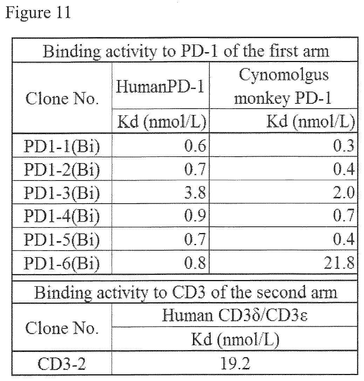

By Biacore (registered trademark) assay using human IgG1-Fc fused human PD-1 extracellular recombinant protein or 6×His tag fused cynomolgus monkey PD-1 extracellular recombinant protein, the binding affinities to the respective PD-1 recombinant proteins of the first arm of the PD-1/CD3 bispecific monoclonal antibodies obtained in Example 8 were evaluated. Note here that Series S Sensor Chip CM5 Sensor Chip (GE Health Care, serial number 29-1049-88) was used for immobilization of the recombinant proteins. Similarly, by Biacore (registered trademark) assay using human IgG1-Fc fused CD3δ/CD3ε extracellular recombinant protein, the binding affinity to CD3 of the second arm of the same antibody was evaluated. Figure 11 shows the binding affinities (Kd value) to PD-1 of the first arms and the binding affinity to CD3δ/CD3ε of the second arm with respect to the respective clones.

### Example 10: Verification of the binding property of the PD-1/CD3 bispecific monoclonal antibody

It was verified that the PD-1/CD3 bispecific monoclonal antibodies obtained in Example 8 specifically, simultaneously bind to PD-1 and CD3, respectively.

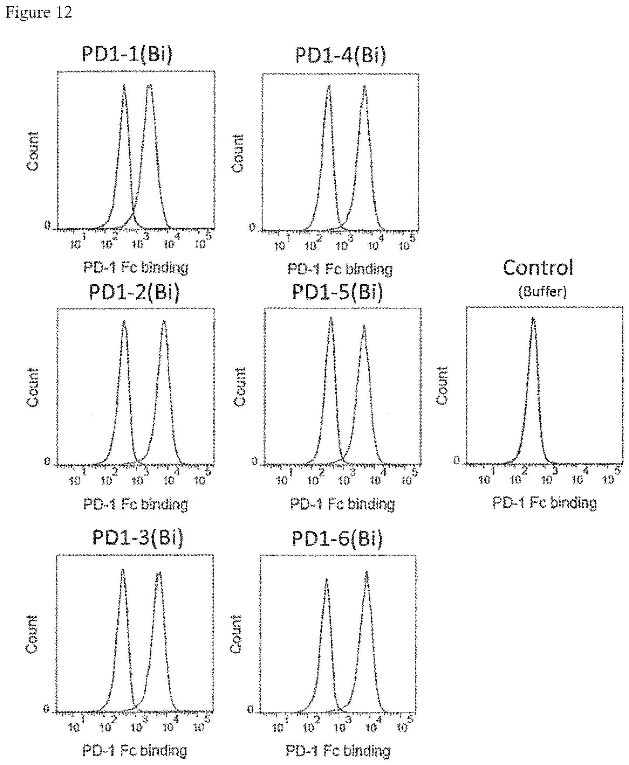

Initially, the clones PD1-1(Bi) to PD1-6(Bi) were added to human PD-1-deficient Jurkat cell lines (human T cell line) expressing human CD3, respectively, and which were incubated on ice for 15 minutes. After those cells were washed, soluble PD-1 recombinant proteins (R&D systems, serial number 1086-PD-050) labeled with 3-fold amount of biotin was added thereto, and which were incubated on ice for 15 minutes. After those cells were washed again, 100 µL of 1.25 µg/mL Alexa Fluor 488-labeled streptavidin (BioLegend, serial number 405235) was added thereto, and which were incubated on ice for 15 minutes. After those cells were further washed, the binding amounts of soluble PD-1 recombinant protein were evaluated by flow cytometry, respectively. Figure 12 shows results thereof in this assay.

All of the clones bound to PD-1 and CD3 simultaneously. Note here that no nonspecific binding in this binding system was detected.

### Example 11: Evaluation of the binding property of the first arm of the PD-1/CD3 bispecific monoclonal antibody

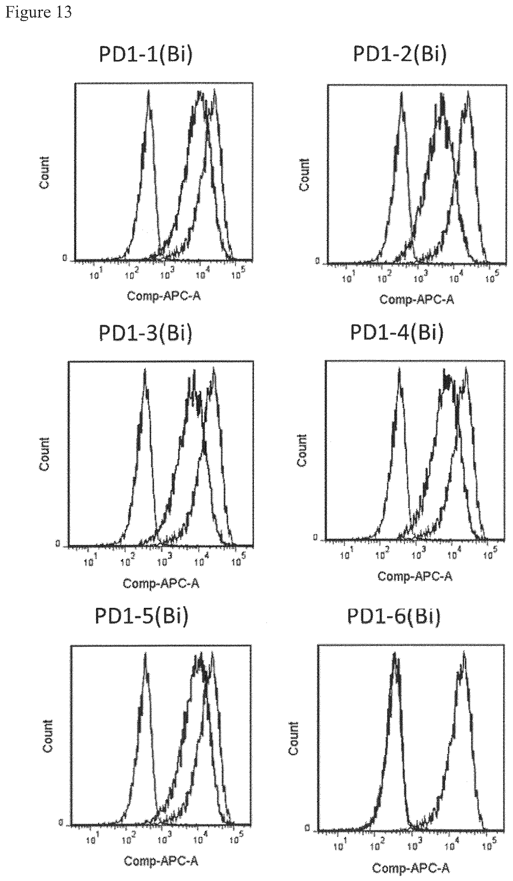

In order to evaluate effects on the PD-1/PD-L1 interaction of the first arms of the PD-1/CD3 bispecific monoclonal antibodies obtained in Example 8, the competitive binding assay with respect to the binding to PD-1 of the bispecific monoclonal antibody clones and soluble PD-L1 recombinant proteins to PD-1 was carried out. Initially, the clones PD1-1(Bi) to PD1-6(Bi) were added to human PD-1 expressing CHO-S cell lines, respectively, and which were incubated on ice for 30 minutes. After those cells were washed, soluble PD-L1 recombinant proteins (R&D systems, serial number 156-B7-100) labeled with 1/20 amount of biotin was added thereto, and which were incubated on ice for 30 minutes. After those cells were washed again, PE-labeled streptavidin (BD Pharmingen, serial number 554061) was added thereto, and which were incubated on ice for 30 minutes. After those cells were further washed, the binding amounts of soluble PD-L1 recombinant proteins were evaluated by flow cytometry. Figure 13 shows results thereof.

Although the clones PD1-1(Bi) to PD1-5(Bi) were in the amount of 20 times more than that of soluble PD-L1 recombinant protein, they allowed the binding of soluble PD-L1 recombinant protein to PD-1. On the other hand, the clone PD1-6(Bi) completely inhibited the binding of soluble PD-L1 recombinant protein to PD-1 at the same conditions.

### Example 12: In vitro suppressive effects of the PD-1/CD3 bispecific monoclonal antibody against activated CD4 T cells

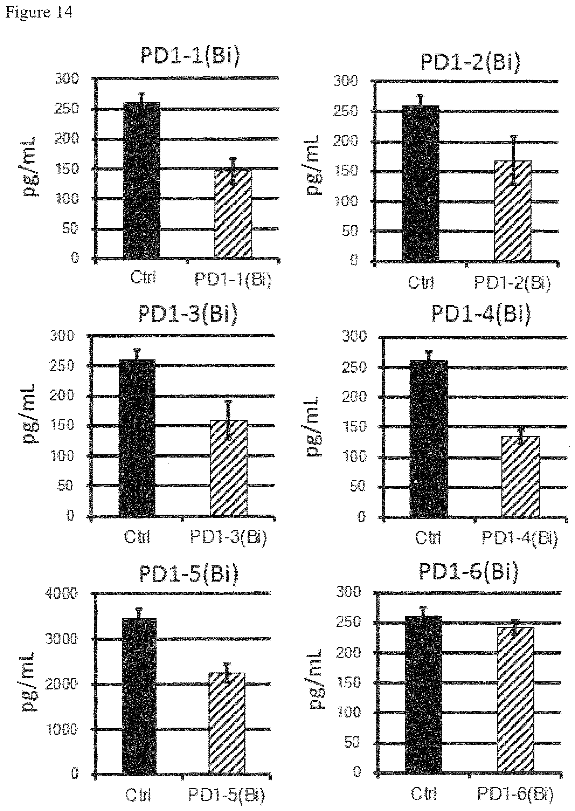

Suppressive effects of activated T cells on IFN-γ production were evaluated using CD4 positive T cells derived from healthy human peripheral blood (LONZA, serial number 2W-200) (human T cells). Human T cells were seeded on cell culture plates on which anti-human TCRVβ8 antibodies (Thermo Scientific, serial number TCR1750) have been solid-phased, anti-human CD28 antibodies (BioLegend, serial number 302923) were added thereto, and subjected to activation treatment for 72 hours. Then, human T cells subjected to activation treatment were cultured overnight in fresh medium containing 100 Units/mL human IL-2 (R&D systems, serial number 202-IL). Furthermore, the collected human T cells were seeded on another cell culture plates on which anti-human TCRVβ8 antibodies have been solid-phased, anti-human CD28 antibodies were added thereto, as well, and then which were subjected to activation treatment again. At this time, the clones PD1-1(Bi) to PD1-6(Bi) were added thereto, respectively, and then IFN-γ contained in the culture supernatant 96 hours after reactivation treatment was quantified by ELISA (pg/mL). Figure 14 shows results thereof. Suppressive activities against IFN-γ production of the clones PD1-1(Bi) to PD1-5(Bi) were confirmed. Suppressive activity against IFN-γ production of the clone PD1-6(Bi) trends to decrease more than other clones. An antibody obtained by immunization of tetanus toxoid by the same technique as that to obtain the first arm, which is a non-specific antibody, was used as a control antibody.

### Example 13: In vivo effects of PD-1/CD3 bispecific monoclonal antibody on experimental allergic encephalomyelitis mouse model (EAE model)

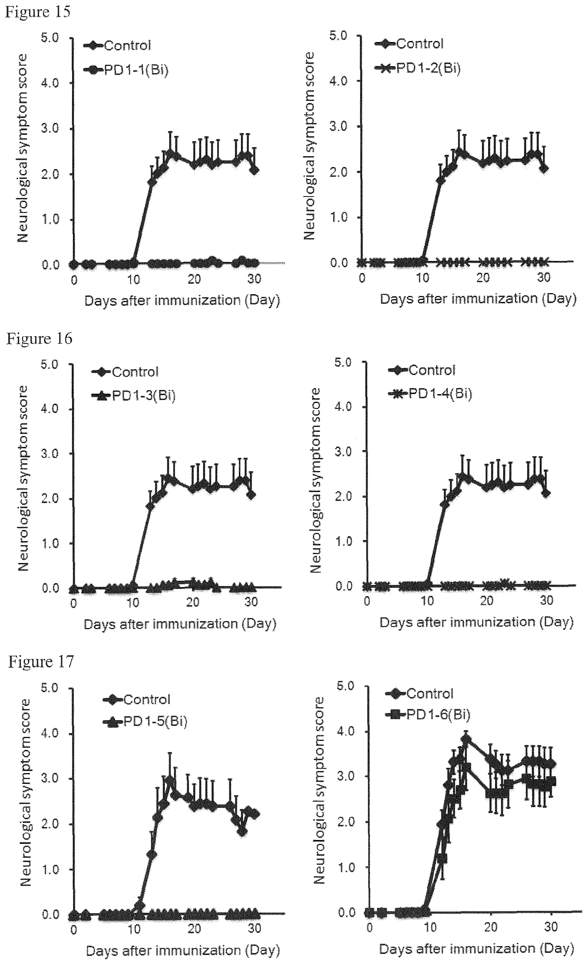

*In vivo* effects of the PD-1/CD3 bispecific monoclonal antibody of the present invention were evaluated in the EAE model using human CD3ε/human PD-1 knock-in C57BL/6 mice in which CD3ε gene and PD-1 gene have been substituted with human CD3ε gene and human PD-1 gene, respectively. Killed mycobacterium tuberculosis H37Ra (BD Biosciences, serial number 231141) and incomplete Freund's adjuvant (BD Biosciences, serial number 263910) were mixed with each other to prepare a complete Freund's adjuvant (CFA) containing 4 mg/mL killed mycobacterium tuberculosis H37Ra. 1 mg/mL MOG peptide (ANASPEC, serial number AS-60130) and the equal amount of CFA were mixed with each other to prepare emulsion as an eliciting agent of the EAE model. 200 µL of the eliciting agent was subcutaneously administered to tail head of the same C57BL/6 mice. On the day of immunization and on day 2, 200 µL of 1 µg/mL of pertussis toxin (SIGMA-ALDRICH, serial number P7208) were administered in tail vein, respectively. Next, on day 6 and 7 since immunization, 2 mg/kg of clones the PD1-1 (Bi) to PD1-6 (Bi) were intraperitoneally administered to the same C57BL/6 mice once a day, respectively. The neurological symptoms after immunization were evaluated in accordance with the method of Onuki, et al. (Onuki M,et al., Microsc Res Tech 2001; 52: 731-9). The degrees of neurological symptoms were scored (normal: score 0, tail relaxation: score 1, hind limb partial paralysis: score 2, hind limb paralysis: score 3, forelimb paralysis: score 4 and dying or death: score 5). If a plurality of neurological symptoms were observed, the higher score was employed as the neurological symptom on the evaluation day. The neurological symptom of died mice was scored to 5 until completion of the observation. Figure 15 to 17 show results thereof, respectively. Note here that human CD3ε/human PD-1 knock-in C57BL/6 mice were prepared by mating human CD3ε knock-in mice produced according to the method described in Genesis., 2009 Jun; 47(6): 414-22 with human PD-1 knock-in mice by a well-known method.

In the EAE model, the onset of neurological symptoms was remarkably suppressed with respect to any of the clones PD1-1 to PD1-5(Bi), but significant suppressive effect was not observed with respect to the clone PD1-6(Bi).

### Example 14: Evaluation of in vitro effect of PD-1/CD3 bispecific monoclonal antibody on cytokine release from human peripheral blood mononuclear cells

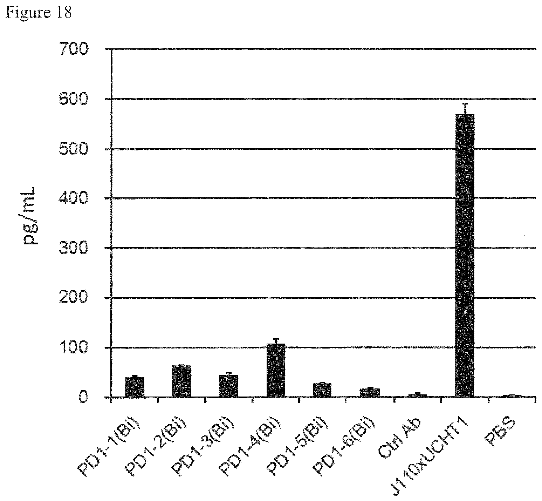

For the purpose of analyzing the cytokine releasing activity of the PD-1/CD3 bispecific monoclonal antibody, an experiment in which the PD-1/CD3 bispecific monoclonal antibody clones of the present invention or the PD-1/CD3 bispecific scDb (J110×UCHT1) disclosed in Patent Literature 2 are added to human peripheral blood mononuclear cells (hereinafter, referred to as "human PBMC") was carried out. The respective clones of the PD-1/CD3 bispecific monoclonal antibodies and J110×UCHT1 were added to human PBMC (LONZA, serial number CC-2702) such that concentrations thereof were 30 µg/mL and 10 µg/mL, respectively, and then which were cultured for 24 hours. After culture for 24 hours, IL-2 contained in culture supernatants thereof was quantified by multiplex immunoassay (BIO-RAD, serial number M50000007A). Figure 18 shows results thereof. Note here that the amount of IL-2 production (pg/mL) in this figure are presented by mean value ± standard error (N = 3).

J110×UCHT1 remarkably induced IL-2 production. However, IL-2 productions with respect to any clone of the PD-1/CD3 bispecific monoclonal antibodies were low.

### Example 15: Evaluation of cross-competitive property of PD1-5(Bi) for binding to PD-1 of the respective clones of the PD-1/CD3 bispecific monoclonal antibodies

Cross-competition assay was conducted to evaluate the cross-competitive properties of the PD1-5(Bi) for binding to PD-1 of the respective clones PD1-1(Bi) to PD1-5(Bi) as the PD-1/CD3 bispecific monoclonal antibody, obtained in Example 8.

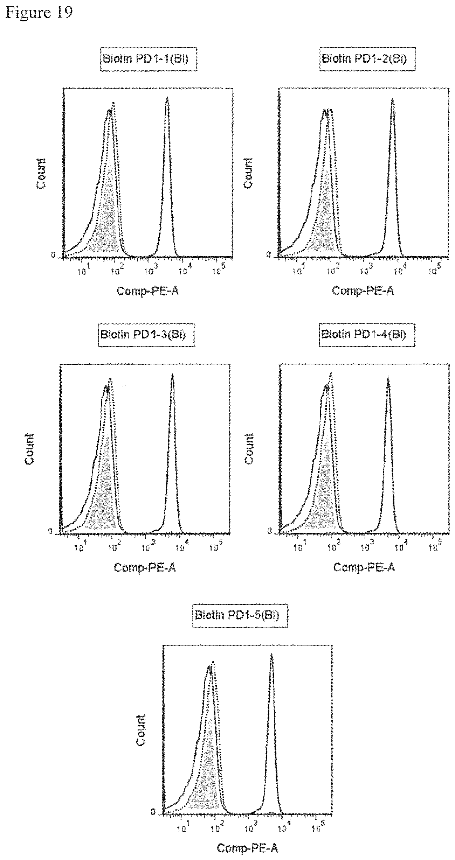

Initially, the clone PD1-5(Bi) was added to human PD-1-expressing CHO-S line cells, then which were incubated on ice for 20 minutes. Further, 1/100 amount of the biotin-labeled clones PD1-1(Bi) to PD1-5(Bi) compared to that of the already-added clone PD1-5(Bi) were added thereto, then which were incubated on ice for 20 minutes. Those cells were washed, PE-labeled streptavidin (BD Pharmingen, model number 554061) was added thereto, and then were incubated on ice for 20 minutes. After washing those cells again, the binding amount of the biotin-labeled clones PD1-1(Bi) to PD1-5(Bi) were measured using flow cytometry. Figure 19 shows results thereof.

It was demonstrated that the clone PD1-5(Bi) can inhibit the respective bindings to PD-1 of the clones PD1-1(Bi) to PD1-5(Bi), and thus that the clone PD1-5(Bi) can cross-compete with their bindings to PD-1.

### Example 16: In vivo effects of the PD-1/CD3 bispecific monoclonal antibody in human peripheral blood mononuclear cell-transplanted hyperimmune-deficient mice

The PD-1/CD3 bispecific monoclonal antibody of the present invention was administered to hyperimmune-deficient mice (NOD.Cg-PrkdcscidI12rgtm1Wj1/SzJ, hereinafter NSG mice) to which human peripheral blood mononuclear cells (PBMC) have been transplanted. *In vivo* effect on T cell proliferation was evaluated.

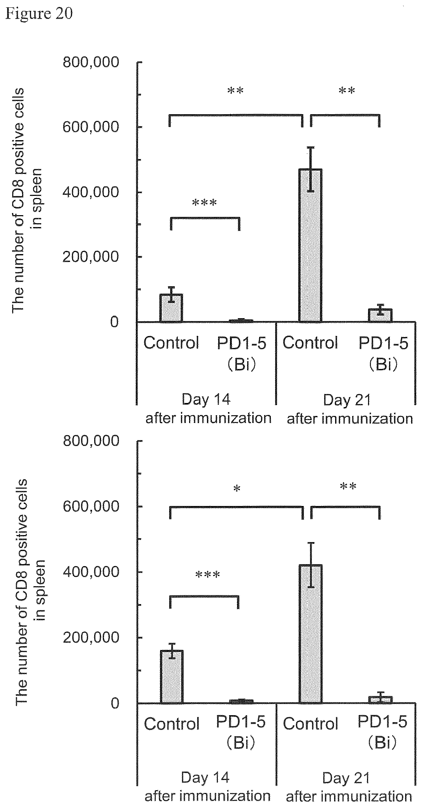

1×10⁷ human PBMC (LONZA, model number CC-2702) per mouse were suspended in DPBS, and which were transplanted into peritoneal cavity of NSG mice. A control antibody or the PD-1/CD3 bispecific monoclonal antibody clone PD1-5 (Bi) was intraperitoneally administered to four NSG mice once a day on 3, 7, 10, 14 and 17 days after transplantation. On 14 or 21 day after transplantation, the same NSG mice were anesthetized by inhalation of isoflurane, and spleens thereof were removed. Cells were isolated from spleens using 70 µm cell strainer (BD Falcon). APC-Cy7 fluorescence-labeled anti-CD4 antibody (Biolegend, model no. 300518) or FITC fluorescence-labeled anti-CD8 antibody (Biolegend, model no. 301006) was added to the prepared cell suspension. The number of human CD4 positive cells and human CD8 positive cells were measured using flow cytometer (BD Biosciences, model BD LSR Fortessa X-20) and analysis software FlowJo (Biolegend, Model No. 300518), respectively. Figure 20 shows results thereof.

On 14 and 21 days after human PBMC transplantation, the PD1-5 (Bi) administration group significantly reduces the number of human CD4 positive cells and human CD8 positive cells in spleen, compared to the control antibody administration group, and the clone PD1-5 (Bi) showed an inhibitory effect against the increase in the number of cells.

### Example 17: In vivo effects of the PD-1/CD3 bispecific monoclonal antibody in the mouse antibody production model

The PD-1/CD3 bispecific monoclonal antibody of the present invention was administered to human CD3ε/human PD-1 knock-in C57BL/6 mice prepared in Example 13, and *in vivo* effects on antibody production was evaluated.

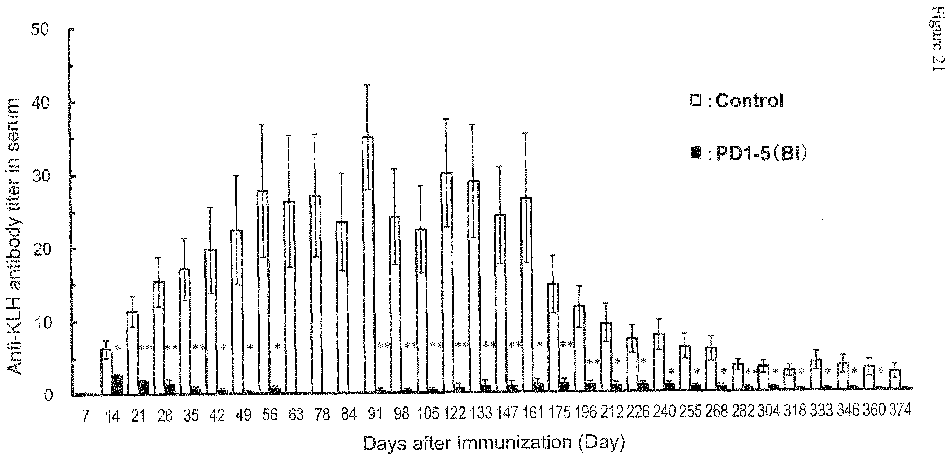

200 µg/mL KLH solution prepared by dissolving Keyhole limpet hemocyanin (Thermo Scienctific, Model No. 77600, hereinafter KLH) with DPBS was further mixed with equal volume of ALUM (Cosmo Bio, Model No. LG-6000) for 30 minutes at room temperature, and which was used as adjuvant. The same mice were intraperitoneally immunized with 200µL of this adjuvant to perform immunization. A control antibody or the PD-1/CD3 bispecific monoclonal antibody clone PD1-5 (Bi) was intraperitoneally administered once a day on 7 and 8 days after immunization. From 7 to 374 days after immunization, 50 to 80 µL of blood was collected from mouse tail vein to prepare serum. The anti-KLH antibody (IgG₁) titer in serum was measured by ELISA. That is, anti-KLH antibody bound to immobilized KLH was detected using HRP-labeled anti-mouse IgG₁ antibody (Bethyl Laboratories, model number A90-105P-38). Figure 21 shows results thereof.

PD1-5 (Bi) showed a suppressive effect on anti-KLH antibody production from the mice. In addition, significant suppressive effects thereof were persisted up to 360 days after immunization.

### Example 18: In vivo effects of the PD-1/CD3 bispecific monoclonal antibody in the spontaneous diabetes model mice

The PD-1/CD3 bispecific monoclonal antibody of the present invention was administered to human CD3ε/human PD-1 knock-in NOD mice which spontaneously develop diabetes, and *in vivo* effects on the change in blood glucose level was evaluated.

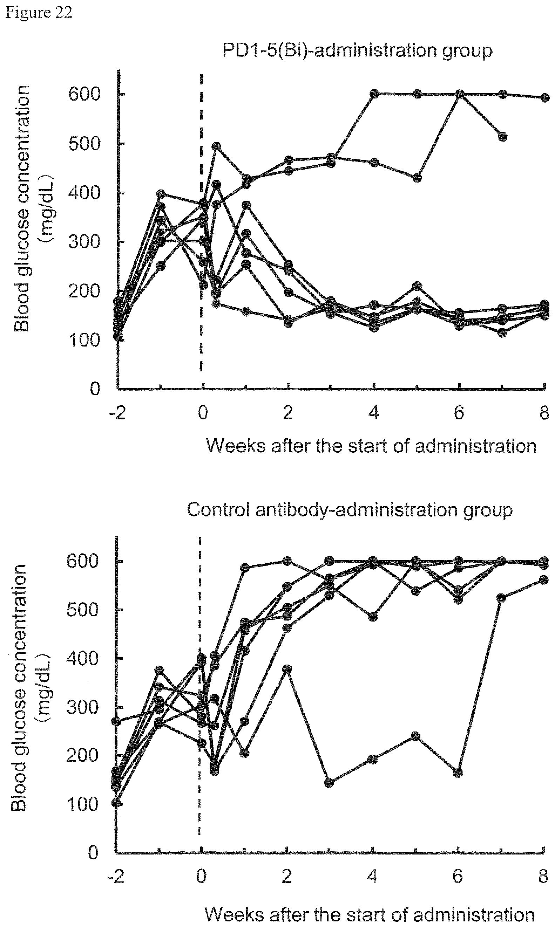

The NOD mice were shaved once a week from 16 ages in weeks with a razor in tail vein, and blood was drawn at 5 to 10 µL, and the concentration of blood glucose was measured using Accucua Aviva (registered trademark) (Roche). A control antibody or the PD-1/CD3 bispecific monoclonal antibody clone PD1-5 (Bi) was intraperitoneally administered for 2 days to 7 individuals with diabetes onset, identified as those in which the concentration of blood glucose was more than 200 mg/dL (high blood sugar area) two or more consecutive times. After the start of administration, blood was collected once a week to measure blood glucose concentration. The concentration of blood glucose, less than 200 mg/dL was taken as a normal blood glucose range. Figure 22 shows results thereof. Note here that the NOD mice were produced by crossing human CD3ε knock-in mice, human PD-1 knock-in mice produced in Example 13, and NOD/ShiJcl mice, according to a known method.

At 7 and 8 weeks after the start of administration, individuals with normal blood glucose range in the PD1-5 (Bi) administration group are significantly more than those in the control antibody administration group, which represents that PD1-5 (Bi) has therapeutic effects in the case of administration after diabetes onset.

### Example 19: Preparation of the PD-1/CD3 bispecific monoclonal antibody with amino acid mutations at the binding site to human FcRn and evaluation of the binding property thereof to FcRn

The antibody clones Mut1, Mut2, Mut3 and Mut4, of which methionine at position 252 under the EU numbering system in the heavy chain constant regions of the PD-1/CD3 bispecific monoclonal antibody clone PD1-5 (Bi) was substituted with glutamic acid, proline, arginine and aspartic acid, respectively, were prepared by methods according to well-known techniques regarding amino acid substitutions. Further, the antibody clone Mut5, of which asparagine at position 434 under the EU numbering system in the heavy chain constant regions of the clone PD1-5 (Bi) was substituted with leucine, and the antibody clone Mut6, of which glutamine at position 438 was substituted with glutamic acid, were prepared by the methods as the above, respectively.

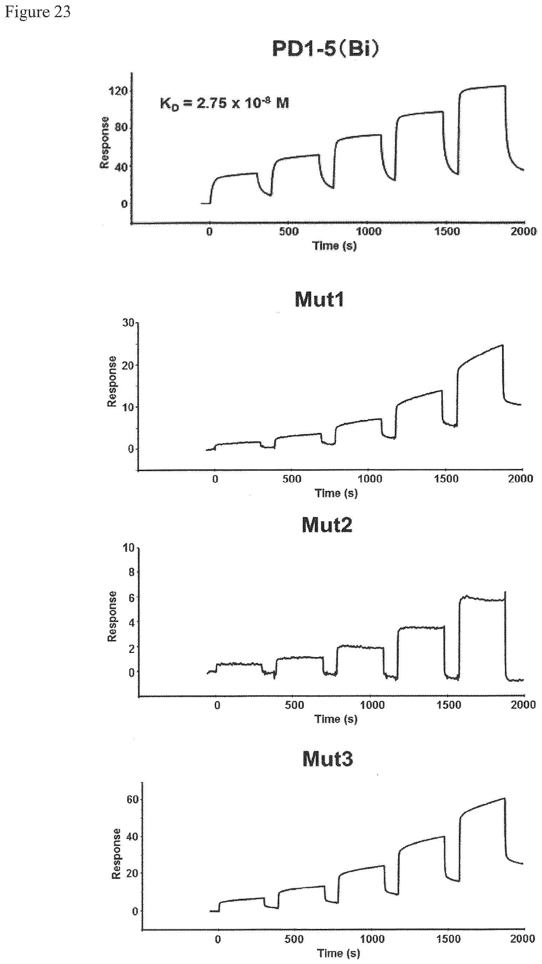

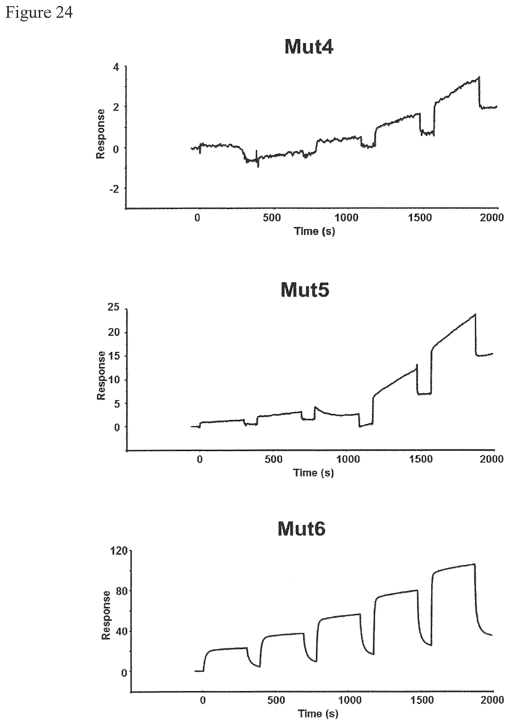

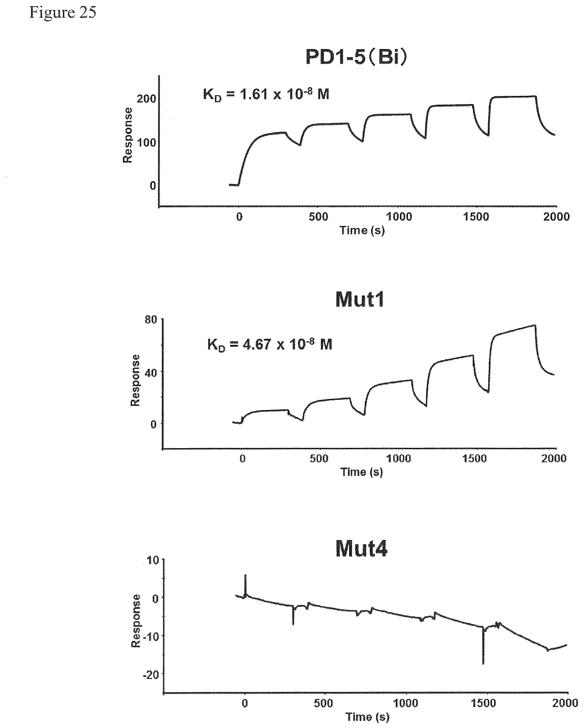

Then, the binding affinities to human FcRn or mouse FcRn of those antibody clones were evaluated using surface plasmon resonance, respectively. Series S Sensor Chip CAP of Biotin CAPture Kit (GE Healthcare, model no. 28-9202-34) was set in Biacore T200 (GE Healthcare), and human FcRn-human B2M (Immunitrack, model no. ITF01) and Murine FcRn-murine B2M (Immunitrack, model no. ITF07) were immobilized thereon, respectively. The binding properties of the PD1-5 (Bi) and Mut1 to Mut6 were evaluated under conditions of pH 6.0, respectively. Figures 23 and 24 show results thereof about the binding properties with human FcRn, and Figure 25 shows those with mouse FcRn.

The respective affinities for human FcRn of the clones Mut1 to Mut6 are lower, compared to that of the PD1-5 (Bi) (dissociation constant (K_{D} value): 2.75 × 10⁻⁸ M), and in particular, those of the clones Mut4 and Mut2 significantly decreased. The respective affinities for mouse FcRn of the clones Mut1 and Mut4 are lower than that of the PD1-5 (Bi) (K_{D} value: 1.61 × 10⁻⁸ M) (K_{D} value of Mut1: 4.67 × 10⁻⁸ M), in particular, that of the clone Mut4 significantly decreased.

### Example 20: Blood kinetics of the PD1/CD3 bispecific monoclonal antibody clone Mut4

*In vivo* blood kinetics of the clone Mut4 was evaluated using human CD3ε/human PD-1 knock-in C57BL/6 mice prepared in Example 13.

At 30 minutes and 1, 2, 4, 8, 24 and 288 hours after administration of the same clone into tail veins of the C57BL/6 mice, tail veins of mice were partially injured with scissors, and approximately 40µL of blood therefrom was collected into heparinized tubes. Blood was collected by centrifugation to prepare plasma, and the plasma concentration of the same clone was measured in the electrochemiluminescence (ECL) method. The half-life was calculated from transition of plasma concentration.

As a result of measurement, the blood half-life of the same clone was 8.0 hours, which was significantly shorter than that of the original PD1-5 (Bi), about 160 hours.

### Example 21: In vivo effects of the PD-1/CD3 bispecific monoclonal antibody in the EAE model

*In vivo* effects of the PD-1/CD3 bispecific monoclonal antibody clone Mut4 was evaluated in the EAE model, prepared according to the method described in Example 13.

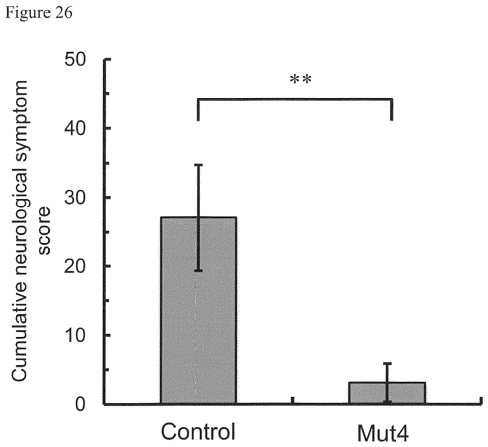

A control antibody or the clone Mut4 was intraperitoneally administered once a day to eight EAE model mice for 5 days up to 6 to 10 days after immunization. The neurological symptoms of mice from the day of immunization were evaluated by the method described in Example 13 according to the method of Onuki M, et al., Microsc Res Tech 2001; 52: 731-9. The cumulative neurological symptom score during the observation period from the day of immunization to 30 day after immunization was counted. Figure 26 shows a result thereof.

The clone Mut4 significantly suppressed neurological symptoms in the EAE model.

### [Industrial Applicability]

The PD-1/CD3 bispecific antibody or antibody fragment thereof of the present invention is useful for preventing, suppressing the progression of symptoms of or the recurrence of and/or treating autoimmune diseases or graft-versus-host diseases (GVHD).

## Claims

1. An IgG₁ bispecific antibody or an antibody fragment thereof, comprising a first arm specifically binding to PD-1 and a second arm specifically binding to CD3, wherein the first arm specifically binding to PD-1 has any one of VH selected from
(A) a VH having
(a) a VH-CDR1 comprising the amino acid sequence set forth in SEQ ID No. 6,
(b) a VH-CDR2 comprising the amino acid sequence set forth in SEQ ID No. 7, and
(c) a VH-CDR3 comprising the amino acid sequence set forth in SEQ ID No. 8,
(B) a VH having
(a) a VH-CDR1 comprising the amino acid sequence set forth in SEQ ID No. 9,
(b) a VH-CDR2 comprising the amino acid sequence set forth in SEQ ID No. 10, and
(c) a VH-CDR3 comprising the amino acid sequence set forth in SEQ ID No. 11,
(C) a VH having
(a) a VH-CDR1 comprising the amino acid sequence set forth in SEQ ID No. 12,
(b) a VH-CDR2 comprising the amino acid sequence set forth in SEQ ID No. 13, and
(c) a VH-CDR3 comprising the amino acid sequence set forth in SEQ ID No. 14,
(D) a VH having
(a) a VH-CDR1 comprising the amino acid sequence set forth in SEQ ID No. 15,
(b) a VH-CDR2 comprising the amino acid sequence set forth in SEQ ID No. 16, and
(c) a VH-CDR3 comprising the amino acid sequence set forth in SEQ ID No. 17, and
(E) a VH having
(a) a VH-CDR1 comprising the amino acid sequence set forth in SEQ ID No. 18,
(b) a VH-CDR2 comprising the amino acid sequence set forth in SEQ ID No. 19, and
(c) a VH-CDR3 comprising the amino acid sequence set forth in SEQ ID No. 20, and
(F) a VL having
(a) a VL-CDR1 comprising the amino acid sequence set forth in SEQ ID No. 26,
(b) a VL-CDR2 comprising the amino acid sequence set forth in SEQ ID No. 27, and
(c) a VL-CDR3 comprising the amino acid sequence set forth in SEQ ID No. 28. and
wherein the second arm specifically binding to CD3 has
(A) a VH having
(a) a VH-CDR1 comprising the amino acid sequence set forth in SEQ ID No. 37,
(b) a VH-CDR2 comprising the amino acid sequence set forth in SEQ ID No. 38, and
(c) a VH-CDR3 comprising the amino acid sequence set forth in SEQ ID No. 39, and
(B) a VL having
(a) a VL-CDR1 comprising the amino acid sequence set forth in SEQ ID No. 26,
(b) a VL-CDR2 comprising the amino acid sequence set forth in SEQ ID No. 27, and
(c) a VL-CDR3 comprising the amino acid sequence set forth in SEQ ID No. 28, and
wherein in two heavy chain constant regions of the IgG₁ antibody, (1) each methionine at position 252 according to the EU numbering system is substituted with glutamic acid, proline, arginine or aspartic acid, (2) each asparagine at position 434 according to the EU numbering system is substituted with leucine and/or (3) each glutamine at position 438 according to the EU numbering system is substituted with glutamic acid, and further one to five arbitrary amino acid residues may be respectively substituted with conservative amino acids thereof in any one or more of CDRs selected from the VH-CDR1, VH-CDR2 and VH-CDR3 in the first arm specifically binding to PD-1, and/or one to five arbitrary amino acid residues may be respectively substituted with conservative amino acids thereof in any one or more of CDRs selected from the VH-CDR1, VH-CDR2 and VH-CDR3 in the second arm specifically binding to CD3.

2. The IgG₁ bispecific antibody or antibody fragment thereof according to claim 1, wherein the first arm specifically binding to PD-1 has any one of VH selected from
(A) the VH having
(a) the VH-CDR1 comprising the amino acid sequence set forth in SEQ ID No. 6,
(b) the VH-CDR2 comprising the amino acid sequence set forth in SEQ ID No. 7, and
(c) the VH-CDR3 comprising the amino acid sequence set forth in SEQ ID No. 8,
(B) the VH having
(a) the VH-CDR1 comprising the amino acid sequence set forth in SEQ ID No. 9,
(b) the VH-CDR2 comprising the amino acid sequence set forth in SEQ ID No. 10, and
(c) the VH-CDR3 comprising the amino acid sequence set forth in SEQ ID No. 11,
(C) the VH having
(a) the VH-CDR1 comprising the amino acid sequence set forth in SEQ ID No. 12,
(b) the VH-CDR2 comprising the amino acid sequence set forth in SEQ ID No. 13, and
(c) the VH-CDR3 comprising the amino acid sequence set forth in SEQ ID No. 14,
(D) the VH having
(a) the VH-CDR1 comprising the amino acid sequence set forth in SEQ ID No. 15,
(b) the VH-CDR2 comprising the amino acid sequence set forth in SEQ ID No. 16, and
(c) the VH-CDR3 comprising the amino acid sequence set forth in SEQ ID No. 17, and
(E) the VH having
(a) the VH-CDR1 comprising the amino acid sequence set forth in SEQ ID No. 18,
(b) the VH-CDR2 comprising the amino acid sequence set forth in SEQ ID No. 19, and
(c) the VH-CDR3 comprising the amino acid sequence set forth in SEQ ID No. 20, and
(F) the VL having
(a) the VL-CDR1 comprising the amino acid sequence set forth in SEQ ID No. 26,
(b) the VL-CDR2 comprising the amino acid sequence set forth in SEQ ID No. 27, and
(c) the VL-CDR3 comprising the amino acid sequence set forth in SEQ ID No. 28, and
wherein the second arm specifically binding to CD3 has
(A) the VH having
(a) the VH-CDR1 comprising the amino acid sequence set forth in SEQ ID No. 37,
(b) the VH-CDR2 comprising the amino acid sequence set forth in SEQ ID No. 38, and
(c) the VH-CDR3 comprising the amino acid sequence set forth in SEQ ID No. 39, and
(B) the VL having
(a) the VL-CDR1 comprising the amino acid sequence set forth in SEQ ID No. 26,
(b) the VL-CDR2 comprising the amino acid sequence set forth in SEQ ID No. 27, and
(c) the VL-CDR3 comprising the amino acid sequence set forth in SEQ ID No. 28.

3. The IgG₁ bispecific antibody or antibody fragment thereof according to claim 1 or 2, wherein (i) the VH of the first arm specifically binding to PD-1 has
(a) the VH-CDR1 comprising the amino acid sequence set forth in SEQ ID No. 6,
(b) the VH-CDR2 comprising the amino acid sequence set forth in SEQ ID No. 7, and
(c) the VH-CDR3 comprising the amino acid sequence set forth in SEQ ID No. 8, and
(ii) the VH of the second arm specifically binding to CD3 has
(a) the VH-CDR1 comprising the amino acid sequence set forth in SEQ ID No. 37,
(b) the VH-CDR2 comprising the amino acid sequence set forth in SEQ ID No. 38, and
(c) the VH-CDR3 comprising the amino acid sequence set forth in SEQ ID No. 39.

4. The IgG₁ bispecific antibody or antibody fragment thereof according to claim 1 or 2, wherein (i) the VH of the first arm specifically binding to PD-1 has
(a) the VH-CDR1 comprising the amino acid sequence set forth in SEQ ID No. 9,
(b) the VH-CDR2 comprising the amino acid sequence set forth in SEQ ID No. 10, and
(c) the VH-CDR3 comprising the amino acid sequence set forth in SEQ ID No. 11, and
(ii) the VH of the second arm specifically binding to CD3 has
(a) the VH-CDR1 comprising the amino acid sequence set forth in SEQ ID No. 37,
(b) the VH-CDR2 comprising the amino acid sequence set forth in SEQ ID No. 38, and
(c) the VH-CDR3 comprising the amino acid sequence set forth in SEQ ID No. 39.

5. The IgG₁ bispecific antibody or antibody fragment thereof according to claim 1 or 2, wherein (i) the VH of the first arm specifically binding to PD-1 has
(a) the VH-CDR1 comprising the amino acid sequence set forth in SEQ ID No. 12,
(b) the VH-CDR2 comprising the amino acid sequence set forth in SEQ ID No. 13, and
(c) the VH-CDR3 comprising the amino acid sequence set forth in SEQ ID No. 14, and
(ii) the VH of the second arm specifically binding to CD3 has
(a) the VH-CDR1 comprising the amino acid sequence set forth in SEQ ID No. 37,
(b) the VH-CDR2 comprising the amino acid sequence set forth in SEQ ID No. 38, and
(c) the VH-CDR3 comprising the amino acid sequence set forth in SEQ ID No. 39.

6. The IgG₁ bispecific antibody or antibody fragment thereof according to claim 1 or 2, wherein (i) the VH of the first arm specifically binding to PD-1 has
(a) the VH-CDR1 comprising the amino acid sequence set forth in SEQ ID No. 15,
(b) the VH-CDR2 comprising the amino acid sequence set forth in SEQ ID No. 16, and
(c) the VH-CDR3 comprising the amino acid sequence set forth in SEQ ID No. 17, and
(ii) the VH of the second arm specifically binding to CD3 has
(a) the VH-CDR1 comprising the amino acid sequence set forth in SEQ ID No. 37,
(b) the VH-CDR2 comprising the amino acid sequence set forth in SEQ ID No. 38, and
(c) the VH-CDR3 comprising the amino acid sequence set forth in SEQ ID No. 39.

7. The IgG₁ bispecific antibody or antibody fragment thereof according to claim 1 or 2, wherein (i) the VH of the first arm specifically binding to PD-1 has
(a) the VH-CDR1 comprising the amino acid sequence set forth in SEQ ID No. 18,
(b) the VH-CDR2 comprising the amino acid sequence set forth in SEQ ID No. 19, and
(c) the VH-CDR3 comprising the amino acid sequence set forth in SEQ ID No. 20, and
(ii) the VH of the second arm specifically binding to CD3 has
(a) the VH-CDR1 comprising the amino acid sequence set forth in SEQ ID No. 37,
(b) the VH-CDR2 comprising the amino acid sequence set forth in SEQ ID No. 38, and
(c) the VH-CDR3 comprising the amino acid sequence set forth in SEQ ID No. 39.

8. The IgG₁ bispecific antibody or antibody fragment thereof according to any one of claims 1 to 7, wherein the FR1, FR2 and FR3 in the VH of the first arm specifically binding to PD-1 correspond to the amino acid sequence encoded by the germ-line V gene IGHV7-4-1 with somatic mutation(s), respectively, and the framework region 4 comprises the amino acid sequence encoded by the germ-line J gene JH6c with somatic mutation(s) (excluding an amino acid sequence included in the VH-CDR3).

9. The IgG₁ bispecific antibody or antibody fragment thereof according to any one of claims 1 to 8, wherein the VH of the first arm specifically binding to PD-1 comprises the amino acid sequence set forth in any one selected from SEQ ID No. 1, SEQ ID No. 2, SEQ ID No. 3, SEQ ID No. 4 and SEQ ID No. 5, or an amino acid sequence having an identity of at least 80% to the amino acid sequence of the same VH

10. The IgG₁ bispecific antibody or antibody fragment thereof according to any one of claims 1 to 9, wherein the VH of the second arm specifically binding to CD3 comprises the amino acid sequence set forth in SEQ ID No. 36, or an amino acid sequence having an identity of at least 80% to the amino acid sequence of the same VH

11. The IgG₁ bispecific antibody or antibody fragment thereof according to claim 1, wherein the VH of the first arm specifically binding to PD-1 comprises the amino acid sequence set forth in any one selected from SEQ ID No. 1, SEQ ID No. 2, SEQ ID No. 3, SEQ ID No. 4 and SEQ ID No. 5, and the VH of the second arm specifically binding to CD3 comprises the amino acid sequence set forth in SEQ ID No. 36.

12. The IgG₁ bispecific antibody or antibody fragment thereof according to claim 1 or 3, wherein the VH of the first arm specifically binding to PD-1 comprises the amino acid sequence set forth in SEQ ID No. 1, and the VH of the second arm specifically binding to CD3 comprises the amino acid sequence set forth in SEQ ID No. 36.

13. The IgG₁ bispecific antibody or antibody fragment thereof according to claim 1 or 4, wherein the VH of the first arm specifically binding to PD-1 comprises the amino acid sequence set forth in SEQ ID No. 2, and the VH of the second arm specifically binding to CD3 comprises the amino acid sequence set forth in SEQ ID No. 36.

14. The IgG₁ bispecific antibody or antibody fragment thereof according to claim 1 or 5, wherein the VH of the first arm specifically binding to PD-1 comprises the amino acid sequence set forth in SEQ ID No. 3, and the VH of the second arm specifically binding to CD3 comprises the amino acid sequence set forth in SEQ ID No. 36.

15. The IgG₁ bispecific antibody or antibody fragment thereof according to claim 1 or 6, wherein the VH of the first arm specifically binding to PD-1 comprises the amino acid sequence set forth in SEQ ID No. 4, and the VH of the second arm specifically binding to CD3 comprises the amino acid sequence set forth in SEQ ID No. 36.

16. The IgG₁ bispecific antibody or antibody fragment thereof according to claim 1 or 7, wherein the VH of the first arm specifically binding to PD-1 comprises the amino acid sequence set forth in SEQ ID No. 5, and the VH of the second arm specifically binding to CD3 comprises the amino acid sequence set forth in SEQ ID No. 36.

17. The IgG₁ bispecific antibody or antibody fragment thereof according to any one of claims 1 to 16, wherein the first arm specifically binding to PD-1 and/or the second arm specifically binding to CD3 have/has the VL comprising the amino acid sequence set forth in SEQ ID No. 25, respectively.

18. An IgG₁ bispecific antibody or an antibody fragment thereof, having a first arm specifically binding to PD-1 and a second arm specifically binding to CD3, wherein
(A) the first arm specifically binding to PD-1 has a VH comprising the amino acid sequence set forth in any one selected from SEQ ID No. 1, SEQ ID No. 2, SEQ ID No. 3, SEQ ID No. 4 and SEQ ID No. 5, and a VL comprising the amino acid sequence set forth in SEQ ID No. 25, and
(B) the second arm specifically binding to CD3 has a VH comprising the amino acid sequence set forth in SEQ ID No. 36, and a VL comprising the amino acid sequence set forth in SEQ ID No. 25, and
wherein in two heavy chain constant regions of the IgG₁ antibody, (1) each methionine at position 252 according to the EU numbering system is substituted with glutamic acid, proline, arginine or aspartic acid, (2) each asparagine at position 434 according to the EU numbering system is substituted with leucine and/or (3) each glutamine at position 438 according to the EU numbering system is substituted with glutamic acid.

19. An IgG₁ bispecific antibody or an antibody fragment thereof, having a first arm specifically binding to PD-1 and a second arm specifically binding to CD3, wherein the first arm specifically binding to PD-1 cross-competes for (1) the binding to PD-1 with the first arm specifically binding to PD-1 having a VH comprising the amino acid sequence set forth in any one selected from SEQ ID No. 1, SEQ ID No. 2, SEQ ID No. 3, SEQ ID No. 4 and SEQ ID No. 5, and a VL comprising the amino acid sequence set forth in SEQ ID No. 25 or (2) the binding to PD-1 with a variable region of a monoclonal antibody specifically binding to PD-1 having the same VH and VL, and
wherein in two heavy chain constant regions of the IgG₁ antibody, (1) each methionine at position 252 according to the EU numbering system is substituted with glutamic acid, proline, arginine or aspartic acid, (2) each asparagine at position 434 according to the EU numbering system is substituted with leucine and/or (3) each glutamine at position 438 according to the EU numbering system is substituted with glutamic acid.

20. An IgG₁ bispecific antibody or an antibody fragment thereof, having a first arm specifically binding to PD-1 and a second arm specifically binding to CD3, wherein the binding to PD-1 with the first arm specifically binding to PD-1 is cross-competed by (1) the first arm specifically binding to PD-1 having a VH comprising the amino acid sequence set forth in any one selected from SEQ ID No. 1, SEQ ID No. 2, SEQ ID No. 3, SEQ ID No. 4 and SEQ ID No. 5, and a VL comprising the amino acid sequence set forth in SEQ ID No. 25 or (2) a variable region of a monoclonal antibody specifically binding to PD-1 having the same VH and VL, and
wherein in two heavy chain constant regions of the IgG₁ antibody, (1) each methionine at position 252 according to the EU numbering system is substituted with glutamic acid, proline, arginine or aspartic acid, (2) each asparagine at position 434 according to the EU numbering system is substituted with leucine and/or (3) each glutamine at position 438 according to the EU numbering system is substituted with glutamic acid.

21. The IgG₁ bispecific antibody or antibody fragment thereof according to claim 19 or 20, wherein the second arm specifically binding to CD3 cross-competes for (1) the binding to CD3 with the second arm specifically binding to CD3 having a VH comprising the amino acid sequence set forth in SEQ ID No. 36, and a VL comprising the amino acid sequence set forth in SEQ ID No. 25, or (2) the binding to CD3 with a variable region of a monoclonal antibody specifically binding to CD3 having the same VH and VL.

22. The IgG₁ bispecific antibody or antibody fragment thereof according to any one of claims 1 to 21, wherein the first arm specifically binding to PD-1 allows the interaction between PD-1 and PD-L1.

23. The IgG₁ bispecific antibody or antibody fragment thereof according to any one of claims 1 to 22, wherein cytokine production during administration or within 24 hours after administration is sufficiently reduced.

24. The IgG₁ bispecific antibody or antibody fragment thereof according to claim 23, wherein the cytokine is at least IL-2, IFN-γ or TNF-α.

25. The IgG₁ bispecific antibody or antibody fragment thereof according to any one of claims 1 to 24, wherein the binding to Fc receptor of the IgG₁ bispecific antibody is eliminated or attenuated.

26. The IgG₁ bispecific antibody or antibody fragment thereof according to claim 25, wherein in two heavy chain constant regions of the IgG₁ antibody, each leucine at position 235 according to the EU numbering system is substituted with glycine, and/or each glycine at position 236 is substituted with arginine.

27. The IgG₁ bispecific antibody or antibody fragment thereof according to any one of claims 1 to 26, wherein in a constant region of the heavy chain having the VH of the first arm specifically binding to PD-1, leucine at position 351 according to the EU numbering system is substituted with lysine and threonine at position 366 is substituted with lysine, and in a constant region of the heavy chain having the VH of the second arm specifically binding to CD3, leucine at position 351 is substituted with aspartic acid and leucine at position 368 is substituted with glutamic acid.

28. The IgG₁ bispecific antibody or antibody fragment thereof according to any one of claims 1 to 26, wherein in a constant region of the heavy chain having the VH of the first arm specifically binding to PD-1, leucine at position 351 according to the EU numbering system is substituted with aspartic acid, and leucine at position 368 is substituted with glutamic acid, and in a constant region of the heavy chain having the VH of the second arm specifically binding to CD3, leucine at position 351 is substituted with lysine, and threonine at position 366 is substituted with lysine.

29. The IgG₁ bispecific antibody or antibody fragment thereof according to any one of claims 1 to 28, wherein in two heavy chain constant regions of the IgG₁ bispecific antibody, each lysine at position 447 according to the EU numbering system is deleted.

30. The IgG₁ bispecific antibody or antibody fragment thereof according to any one of claims 1 to 29, wherein in two heavy chain constant regions of the IgG₁ antibody, methionine at position 252 according to the EU numbering system is substituted with aspartic acid.

31. The IgG₁ bispecific antibody or antibody fragment thereof according to any one of claims 1 to 30, wherein the binding to neonatal Fc receptor is eliminated or attenuated.

32. The IgG₁ bispecific antibody or antibody fragment thereof according to any one of claims 1 to 31, wherein the half-life in blood of the IgG₁ antibody described in any one of claims 1 to 31 is shortened compared to the original antibody without the same amino acid substitution(s).

33. The IgG₁ bispecific antibody or antibody fragment thereof according to any one of claims 1 to 32, wherein the half-life in blood of the IgG₁ antibody described in any one of claims 1 to 32 is shortened at least 50%, at least 60%, at least 70%, at least 80% or at least 90%, compared to the original antibody without the same amino acid substitution(s).

34. The IgG₁ bispecific antibody or antibody fragment thereof according to any one of claims 1 to 33, wherein the heavy chain having the VH of the first arm specifically binding to PD-1 has a heavy chain constant region comprising the amino acid sequence set forth in any one selected from SEQ ID No. 42, SEQ ID No. 43, SEQ ID No. 44, SEQ ID No. 45, SEQ ID No. 46 and SEQ ID No. 47.

35. The IgG₁ bispecific antibody or antibody fragment thereof according to any one of claims 1 to 34, wherein the heavy chain having the VH of the second arm specifically binding to CD3 has a heavy chain constant region comprising the amino acid sequence set forth in any one selected from SEQ ID No. 48, SEQ ID No. 49, SEQ ID No. 50, SEQ ID No. 51, SEQ ID No. 52 and SEQ ID No. 53.

36. The IgG₁ bispecific antibody or antibody fragment thereof according to any one of claims 1 to 35, wherein the light chain having the VL of the first arm specifically binding to PD-1 and/or the light chain having the VL of the second arm specifically binding to CD3 have/has a light chain constant region comprising the amino acid sequence set forth in SEQ ID No. 29.

37. An IgG₁ bispecific antibody or an antibody fragment thereof, having a first arm specifically binding to PD-1 and a second arm specifically binding to CD3, wherein
(A) a heavy chain having the VH of the first arm specifically binding to PD-1 has a VH comprising the amino acid sequence set forth in any one selected from SEQ ID No. 1, SEQ ID No. 2, SEQ ID No. 3, SEQ ID No. 4 and SEQ ID No. 5 and a heavy chain constant region comprising the amino acid sequence set forth in any one selected from SEQ ID No. 42, SEQ ID No. 43, SEQ ID No. 44, SEQ ID No. 45, SEQ ID No. 46 and SEQ ID No. 47,
(B) a light chain having the VL of the first arm specifically binding to PD-1 has a VL comprising the amino acid sequence set forth in SEQ ID No. 25 and a light chain constant region comprising the amino acid sequence set forth in SEQ ID No. 29,
(C) a heavy chain having the VH of the second arm specifically binding to CD3 has a VH comprising the amino acid sequence set forth in SEQ ID No. 36 and a heavy chain constant region comprising the amino acid sequence set forth in any one selected from SEQ ID No. 48, SEQ ID No. 49, SEQ ID No. 50, SEQ ID No. 51, SEQ ID No. 52 and SEQ ID No. 53, and
(D) a light chain having the VL of the second arm specifically binding to CD3 has a VL comprising the amino acid sequence set forth in SEQ ID No. 25 and a light chain constant region comprising the amino acid sequence set forth in SEQ ID No. 29.

38. A pharmaceutical composition comprising the IgG₁ bispecific antibody or antibody fragment thereof, having the first arm specifically binding to PD-1 and the second arm specifically binding to CD3, selected from any one of claims 1 to 37, and a pharmaceutically acceptable carrier.

39. An agent for preventing, suppressing the progression of symptoms of or the recurrence of and/or treating autoimmune diseases, comprising the IgG₁ bispecific antibody or antibody fragment thereof, having the first arm specifically binding to PD-1 and the second arm specifically binding to CD3, selected from any one of claims 1 to 37 as an active ingredient.
